(19) Europäisches Patentamt
European Patent Office
Office européen des brevets

(11) **EP 4 471 067 A1**

# (12) EUROPEAN PATENT APPLICATION

(43) Date of publication:
**04.12.2024 Bulletin 2024/49**

(21) Application number: **23176793.0**

(22) Date of filing: **01.06.2023**

(51) International Patent Classification (IPC):
***C07K 16/44*** *(2006.01)* ***A61K 39/00*** *(2006.01)*

(52) Cooperative Patent Classification (CPC):
**C07K 16/44; A61K 39/4611; A61K 39/4631; A61K 39/464412; A61K 39/4646;** A61K 2239/11; A61K 2239/23; A61K 2239/24; C07K 2317/14; C07K 2317/20; C07K 2317/569; C07K 2317/92

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Miltenyi Biotec B.V. & Co. KG**
**51429 Bergisch Gladbach (DE)**

(72) Inventors:
- **SYLVANDER, Elise**
  **1090 Vienna (AT)**
- **SALZER, Benjamin**
  **1090 Vienna (AT)**
- **ZAJC, Charlotte**
  **1180 Vienna (AT)**
- **LEHNER, Manfred**
  **1090 Vienna (AT)**
- **TRAXLMAYR, Michael**
  **1180 Vienna (AT)**

(74) Representative: **Biervert, Christian**
**c/o Miltenyi Biotec B.V. & Co. KG**
**Corporate Legal Department**
**Friedrich-Ebert-Straße 68**
**51429 Bergisch Gladbach (DE)**

Remarks:
The complete document including Reference Table(s) and the Sequence Listing(s) can be downloaded from the EPO website

## (54) A CHEMICALLY INDUCIBLE HETERODIMERIZING SYSTEM AND A METHOD FOR GENERATION THEREOF

(57) The present invention provides a method of creating a chemically-induced heterodimerizing system having three different components that form a ternary complex by amendment of a chemically induced homodimerizing system, wherein said chemically induced homodimerizing system comprises two components for the homodimerization, wherein the antigen binding domain comprising SEQ ID NO:1 (ABO) is the first component and a small molecule such as caffeine is the second component of the homodimerization, and wherein said ABO and said small molecule form a complex of ABO/ABO/small molecule. Heterodimerizing systems obtained by said method are also disclosed herein.

D
8 NNK library
2 x Positive sort
1 x Negative sort
1 x Positive sort
Low
High
1 x Negative sort
3 x Positive sort
- 1 x Stability sort
48°C heat shock, 10min
- 1 x Affinity sort
20nM V104D
Sequencing
Sequencing

Figure 2.

EP 4 471 067 A1

Processed by Luminess, 75001 PARIS (FR)

## Description

### Field of the Invention

The present invention generally relates to the field of the generation of a chemically-induced heterodimerizing system, in particular to the generation of a chemically-induced heterodimerizing system starting from an existing homodimerizing system, wherein the chemically induced heterodimerizing system uses derivatives of a caffeine-binding nanobody and a small molecule such as a derivative of xanthine, e.g. caffeine as the non-protein component.

### Background of the invention

Chemically induced dimerizing systems are powerful tools for dose and temporal control over protein-protein interactions. Examples for the use of such chemically induced dimerizing systems are the regulation of transcriptional activation by synthetic transcription factors or the safety mechanism in the immune cell therapy that uses a chimeric antigen receptor (CAR) expressed on the surface of genetically engineered immune cells.

A widely used example of the chemically induced dimerizing technology is the FKBP12/FRB dimerization system (Rivera et al., Nature medicine. 1996;2(9):1028-32; Choi et al., Science. 1996;273(5272):239-42). FKBP12 and FRB are human derived proteins which heterodimerize in the presence of the immunosuppressive small molecule drug, rapamycin. Fusion of FKBP12 to a DNA binding domain and fusion of FRB to a transcriptional activation domain produces a system where addition of rapamycin activates gene expression by recruiting the transcriptional activation domain into proximity of the promoter DNA (Rivera et al., Nature medicine. 1996;2(9):1028-32). In WO2014127261A1 a regulatable CAR system is disclosed using e.g. FKBP and FKBP-rapamycin binding domain (FRB) as dimerizing domains and a rapalog as a dimerizing agent.

Existing systems for small molecule-induced heterodimerization of proteins are not well suited for clinical application due to the strong side effects and/or unfavorable pharmacological properties of the small molecules.

Ladenson, R. C. et al. (2006, Analytical Chemistry 78, 4501-4508) isolated and characterized a thermally stable caffeine-specific single domain antibody fragment from llama (i.e. acVHH) that was used as a chemically induced homodimerization system (Bojar, D, et al, 2018, Nat. Commun. 9, 2318). That is, caffeine induces the homodimerization of this acVHH nanobody.

Such a homodimerization system has the disadvantage of limited applicability if two different components such as effector molecules shall be dimerized with the system. WO2018213848A1 discloses a system comprising (a) a first chemically-induced dimer (CID) component comprising (i) a first binding moiety capable of interacting with a small molecule to form a complex between the first CID component and the small molecule; and (ii) a first adapter moiety linked to the first binding moiety, or a first nucleic acid encoding polypeptide components of the first CID component; and (b) a second CID component comprising (i) a second binding moiety that specifically binds to the complex between the small molecule and the first CID component; and (ii) a second adapter moiety linked to the second binding moiety, or a second nucleic acid encoding polypeptide components of the second CID component, wherein the second binding moiety specifically binds to a site of the complex comprising at least a portion of the small molecule and a portion of the first binding moiety. WO2018213848A1 defines a "small molecule" broadly and vaguely as an organic compound, including an organometallic compound, of a molecular weight less than about 2 kDa.

WO 2017/070554 discloses a method of creating a chemically-induced dimerizing protein system comprising: selecting a metatype antibody binding domain (AB2) that binds a portion of a first binding domain (AB1), but only when AB1 is bound to a small molecule to form an AB 1/small molecule complex. Numerous small molecules can be selected for use with the system.

There is a need in the art for an improved or alternative method for the generation of a chemically-induced heterodimerization system from a chemically induced homodimerization system and/or alternative or improved components of such a chemically induced heterodimerization system developed from a chemically induced homodimerization system for use in a switch system e.g. in engineered immune cells.

### Brief description of the invention

The inventors surprisingly found a method that allows, based on an existing chemically induced homodimerization system which uses caffeine and a caffeine binding nanobody (Franco, E. J. et al., 2010, Journal of Chromatography B 878, 177-186, Bojar, D, et al, 2018, Nat. Commun. 9, 2318; WO2006/036882A2, Lesne, J, et al, 2019, Scientific reports 9:1840), to develop a system for a chemically induced heterodimerization that can be controlled using a clinically applicable small molecule, i.e. a derivative of xanthine such as caffeine or theophylline.

The existing thermally stable caffeine-specific single domain antibody fragment from llama used for homodimerization is herein also referred to as acVHH-WT (or in short "WT") and comprises SEQ ID NO: 1.

To generate a heterodimeric switch from this homodimeric dimerizing system, the inventors started by designing several different acVHH-WT variants with charged amino acids introduced into the interaction surface between the two nanobody domains under the assumption that this would abolish homodimerization. After expressing and analyzing those variants, the inventors exemplarily chose acVHH-V104D (or in short "V104D") having SEQ ID NO:2 as a final candidate due to its high expression level, monomeric profile, and resistance to aggregation. For the second half of the switch the inventors used a yeast display-based directed evolution approach to engineer acVHH-WT variants that would specifically bind to acVHH-V104D in the presence of caffeine. They started by randomizing eight amino acids (acVHH-8NNK; SEQ ID NO:3, wherein positions denoted with "X" represent amino acid positions that were randomly mutated using NNK codons) on a loop in close proximity to the V104D mutation on the opposing nanobody and used yeast-display selections to isolate acVHH-WT mutants that specifically fit to the acVHH-V104D variant. After sequential rounds of positive and negative selection, they obtained four mutants termed acVHH-M1 (or in short "M1") having SEQ ID NO:4, acVHH-M2 (or in short "M2") having SEQ ID NO:5, dfM1-1 having SEQ ID NO:6 and dfM1-2 having SEQ ID NO:7, respectively, all of which showed high affinity in the nanomolar range towards acVHH-V104D and - importantly - these interactions were highly dependent on the presence of caffeine. Additionally, the inventors confirmed the functionality of these chemically induced heterodimerization systems in the reducing environment of the cytoplasm using the NanoBiT® complementation assay (Promega). Moreover, the inventors also demonstrated the lack of (or strongly reduced) homodimerization of acVHH-V104D in the presence of caffeine when compared with acVHH-WT, thus representing a crucial advantage over the previously existing chemically induced homodimerization system based on acVHH-WT.

The major advantage of this novel method for the generation of a heterodimerization system is the heterodimerizing small molecule (the dimerizer), i.e. a derivative of xanthine such as caffeine, theobromine or theophylline, which has highly advantageous pharmacological properties and allows cost-effective control of protein heterodimerization. The invention disclosed herein provides a method for the generation of e.g. a caffeine dependent heterodimerization system and specific components obtained by said method, wherein the two proteins, i.e. the modified nanobodies derived from the parental nanobody acVHH-WT that can bind to the small molecule, e.g. caffeine, are fused to an effector molecule, respectively.

The present invention also provides chemically-induced heterodimerizing systems having specific sequences for the first and second components of said heterodimerizing system as disclosed herein which have been generated by the method as disclosed herein.

Upon heterodimerization induced by caffeine, the effector molecules of both modified nanobodies may exert an effect, e.g. to the cell in which they are expressed, that is not efficiently provided, when both effector molecules are not connected via caffeine.

This allows for, e.g., controlling the function of CAR immune cells in a subject after administration of caffeine, if a CAR such as a split CAR comprises the two components of the heterodimerizing system obtained by the method as disclosed herein.

Compared to the previously existing homodimeric caffeine-dependent switch, the heterodimeric molecular switches generated in this invention greatly expand the range of applications, since in most engineered systems a heterodimerization needs to be controlled. Moreover, another critical advantage of the heterodimeric molecular switches disclosed in this invention may be their higher $EC_{50}$ values, i.e., at least 2-fold higher, 4-fold higher or 10-fold higher as compared with the previously existing homodimeric caffeine-dependent switch (acVHH-WT) as determined by yeast surface display titration experiments. That is, higher concentrations of caffeine may be required to efficiently trigger the switches disclosed herein. Drinking one cup of coffee results in maximum plasma caffeine levels of ~10 μM (Teekachunhatean, S. et al, 2013, ISRN Pharmacol. 2013, 147238). However, in the absence of coffee, tea and chocolate consumption for 21 days, plasma levels of caffeine were below the detection limit (~500 nM) of the respective method (Robertson, D. et al., 1978, N Engl J Med 1978; 298:181-186). In another study, the authors reported that caffeine plasma levels of nonhabitual coffee drinkers after coffee abstinence for at least 16 hours were 1.9±1.6 μmol/L (Corti, R. et al., 2002, Circulation 106:2935-2940). Thus, with highly sensitive switches such as the previously described caffeine-dependent homodimeric switch, those little steady state levels of caffeine in the general population would constantly trigger the switch to a certain extent (depending on the concentration). In contrast, the use of the method as disclosed herein may lead to increased $EC_{50}$ values of the heterodimeric switches, which may minimize this steady state activation in the absence of coffee, tea and chocolate consumption. Thus, the heterodimeric system may be less leaky when being applied in humans, thus providing a further crucial advantage compared with the previously existing homodimeric switches.

**Brief description of the drawings**

Figure 1A-E: Development of a first antigen binding domain (AB 1) with a first modification according to the present invention.

Figure 2A-H: Development of a second antigen binding domain (AB2) with a second modification according to the present invention.

Figure 3A-O: Development of a disulfide-free second antigen binding domain (AB2) with a second modification according to the present invention, which improves intracellular function in the reducing environment of the cytoplasm. Moreover, this Figure also shows a detailed biochemical characterization of the engineered chemically-induced heterodimerizing systems.

Figure 4A-D: Integration of the engineered chemically-induced heterodimerizing systems into CAR molecules for small molecule-mediated regulation of CAR T cell activity.

**Detailed description of the invention**

In a first aspect the present invention provides a method of creating a chemically-induced heterodimerizing system having three different components that form a ternary complex by amendment of a chemically induced homodimerizing system, wherein said chemically induced homodimerizing system comprises two components for the homodimerization, wherein the antigen binding domain comprising SEQ ID NO:1 (AB0) is the first component and a small molecule is the second component of the homodimerization, and wherein said AB0 and said small molecule form a complex of AB0/AB0/small molecule, the method comprising

a) modifying the antigen binding domain AB0
b) selecting a first antigen binding domain (AB 1) with a first modification from modified antigen binding domains of step a), wherein said first modification results in an impairment or a loss of said homodimerization (between two molecules of AB 1 in the presence of said small molecule), thereby creating said AB 1 as the first component of the heterodimerizing system
c) modifying again the antigen binding domain AB0
d) selecting a second antigen binding domain (AB2) with a second modification from modified antigen binding domains of step c), wherein said second modification results in a heterodimerization of AB2 with AB 1 in the presence of the small molecule, and wherein said first modification and said second modification are different modifications, thereby creating said AB2 as the second component of the heterodimerizing system
e) expressing said AB 1 as a fusion protein with a first effector molecule and expressing said AB2 as a fusion protein with a second effector molecule,

wherein said small molecule is the third component of the heterodimerizing system, and wherein the presence of all three components of said heterodimerizing system allows to form a ternary complex of AB 1/AB2/small molecule,
and wherein said small molecule is a derivative of xanthine.

Said method, wherein the impairment or a loss of said homodimerization (between two molecules of AB1 in the presence of said small molecule) is determined by using a protein complementation assay (NanoBiT® complementation assay).

Said method, wherein the impairment or a loss of said homodimerization (between two molecules of AB1 in the presence of said small molecule) is determined by using a protein complementation assay (NanoBiT® complementation assay), and wherein the signal in this protein complementation assay after background subtraction yielded for the homodimerization of AB 1 in the presence of said small molecule is at least 2-fold, at least 5-fold, at least 10-fold, at least 20-fold or at least 50-fold lower compared with the signal in this protein complementation assay after background subtraction yielded for the homodimerization of AB0 in the presence of said small molecule, and wherein said small molecule such as caffeine is administered at a concentration of 10-50 μM.

Said method, wherein said first modification results in a reduced binding between AB 1 and AB0 in the presence of said small molecule, when compared to the binding in said homodimerization (between AB0, AB0 and said small molecule) e.g. in a protein complementation assay (NanoBiT® complementation assay), and wherein said reduction of binding signal is at least 2-fold, at least 10-fold, at least 5-fold, or at least 20-fold.

Said method, wherein said first modification results in a reduced binding between AB 1 and AB0 in the presence of said small molecule, when compared to the binding in said homodimerization (between AB0, AB0 and said small molecule) e.g. in a protein complementation assay (NanoBiT® complementation assay), and wherein said reduction of binding signal is at least 2-fold, at least 10-fold, at least 5-fold, or at least 20-fold, and wherein said small molecule such as caffeine is administered at a concentration of 10-50 μM.

Said method, wherein said second modification results additionally in an impairment or a loss of homodimerization between two molecules of AB2 in the presence of said small molecule. Said method, wherein only the presence of all three components of said heterodimerizing system allows to form a ternary complex of AB1/AB2/small molecule.

Said method, wherein only the presence of all three components of said heterodimerizing system allows form a stable complex, wherein said stable complex is a ternary complex of AB1/AB2/small molecule.

Said method, wherein said ternary complex has an $EC_{50}$ value of 1 μM or higher, 5 μM or higher, 10 μM or higher, 15 μM or higher, 20 μM or higher, or 50 μM or higher, as determined by yeast surface display.

Said method, wherein said heterodimerizing system of AB 1/AB2 has an increased $EC_{50}$ value (i.e. a decreased sensitivity) for the small molecule as compared to said homodimerizing system of AB0/AB0.

Said method, wherein said heterodimerizing system of AB 1/AB2 has an increased $EC_{50}$ value (i.e. a decreased sensitivity) for caffeine as compared to said homodimerizing system of AB0/AB0.

Said method, wherein said heterodimerizing system of AB 1/AB2 has an increased $EC_{50}$ value (i.e. a decreased sensitivity) for theobromine as compared to said homodimerizing system of AB0/AB0.

Said method, wherein said heterodimerizing system of AB 1/AB2 has an increased $EC_{50}$ value for the small molecule as compared to said homodimerizing system of AB0/AB0, wherein said $EC_{50}$ value is increased by at least 2-fold, at least 4-fold, or at least 10-fold.

Said method, wherein said heterodimerizing system of AB1/AB2 has an increased $EC_{50}$ value for caffeine as compared to said homodimerizing system of AB0/AB0, wherein said $EC_{50}$ value is increased by at least 2-fold, at least 4-fold, or at least 10-fold.

Said method, wherein said heterodimerizing system of AB 1/AB2 has an increased $EC_{50}$ value for theobromine as compared to said homodimerizing system of AB0/AB0, wherein said $EC_{50}$ value is increased by at least 2-fold, at least 4-fold, or at least 10-fold.

Said method, wherein said ternary complex of said 3 components may be characterized by a $K_D$ value of 50 μM or below, 5 μM or below, or 500 nM or below, wherein said $K_D$ value defines the affinity between AB 1 and AB2 in the presence of said small molecule.

Said method, wherein the binding between AB1 and the small molecule in the absence of AB2 may be either not detectable by SPR or only may show low affinity with a $K_D$ value above 20 μM, above 40 μM, above 80 μM, above 160 μM, above 300 μM or above 1 mM (as determined by SPR).

Said method, wherein the binding between AB1 and the small molecule in the absence of AB2 may be either not detectable by SPR or only may show low affinity with a $K_D$ value above 20 μM, above 40 μM, above 80 μM, above 160 μM, above 300 μM or above 1 mM as determined by SPR, if the small molecule is caffeine.

Said method, wherein the binding between AB1 and the small molecule in the absence of AB2 and the binding between AB2 and the small molecule in the absence of AB 1 may be either not detectable by surface plasmon resonance (SPR) or only may show low affinity with a $K_D$ value above 20 μM, above 40 μM, above 80 μM, above 160 μM, above 300 μM or above 1 mM as determined by SPR, respectively.

Said method, wherein the binding between AB1 and the small molecule in the absence of AB2 and the binding between AB2 and the small molecule in the absence of AB1 are either not detectable by SPR or only show low affinity with a $K_D$ value above 20 μM, above 40 μM, above 80 μM, above 160 μM, above 300 μM or above 1 mM, respectively, if the small molecule is caffeine.

Said method, wherein the affinity between AB 1 and AB2 in the absence of said small molecule may be at least 5-fold, at least 20-fold, at least 100-fold or at least 500-fold weaker (i.e., higher $K_D$ value) compared to the affinity between AB 1 and AB2 in the presence of said small molecule.

Said method, wherein the binding signal between AB 1 and AB2 obtained in a protein complementation assay (NanoBiT complementation assay) in the presence of said small molecule may be at least 2-fold, at least 5-fold, at least 10-fold, at least 20-fold or at least 50-fold higher compared with the binding signal between AB1 and AB2 obtained in a protein complementation assay (NanoBiT complementation assay) in the absence of said small molecule.

Said method, wherein the binding signal between AB 1 and AB2 obtained in a protein complementation assay (NanoBiT complementation assay) in the presence of 10-50 μM of said small molecule (such as caffeine) may be at least 2-fold, at least 5-fold, at least 10-fold, at least 20-fold or at least 50-fold higher compared with the binding signal between AB 1 and AB2 obtained in a protein complementation assay (NanoBiT complementation assay) in the absence of said small molecule.

Said method, wherein said ternary complex of said 3 components may be characterized by a $K_D$ value of 50 μM or below, 5 μM or below, or 500 nM or below, wherein said $K_D$ value defines the affinity between AB 1 and AB2 in the presence of said small molecule, and wherein the binding between AB1 and the small molecule in the absence of AB2 and the binding between AB2 and the small molecule in the absence of AB 1 may be either not detectable by SPR or only may show low affinity with a $K_D$ value above 20 μM, above 40 μM, above 80 μM, above 160 μM, above 300 μM or above 1 mM, respectively, and wherein the affinity between AB1 and AB2 in the absence of said small molecule may be at least 5-fold, at least 20-fold, at least 100-fold or at least 500-fold weaker (i.e., higher $K_D$ value) compared to the affinity between AB 1 and AB2 in the presence of said small molecule.

Said method, wherein said first modification comprises the amino acid replacement V104D or V104E in SEQ ID NO:1 (AB0), resulting in an antigen binding domain (AB 1) having at least 90% or at least 95% identity with the amino acid sequence as set forth in SEQ ID NO:2, if said amino acid replacement is V104D, or having at least 90% or at least 95% identity with the amino acid sequence as set forth in SEQ ID NO:8, if the amino acid replacement is V104E.

In one embodiment, the invention provides a method of creating (or generation of) a chemically-induced heterodimerizing system having three different components that form a ternary complex by amendment of a chemically induced homodimerizing system, wherein said chemically induced homodimerizing system comprises two components for the homodimerization, wherein the antigen binding domain comprising SEQ ID NO:1 (AB0) is the first component and a small molecule is the second component of the homodimerization, and wherein said AB0 and said small molecule form a complex of AB0/AB0/small molecule, the method comprising

a) modifying the antigen binding domain AB0
b) selecting a first antigen binding domain (AB 1) with a first modification from modified antigen binding domains of step a), wherein said first modification results in an impairment or a loss of said homodimerization (between two molecules of AB 1 in the presence of said small molecule), thereby creating said AB 1 as the first component of the heterodimerizing system
c) modifying again the antigen binding domain AB0
d) selecting a second antigen binding domain (AB2) with a second modification from modified antigen binding domains of step c), wherein said second modification results in a heterodimerization of AB2 with AB 1 in the presence of the small molecule, and wherein said first modification and said second modification are different modifications, thereby creating said AB2 as the second component of the heterodimerizing system
e) expressing said AB 1 as a fusion protein with a first effector molecule and expressing said AB2 as a fusion protein with a second effector molecule,

wherein said small molecule is the third component of the heterodimerizing system, and
wherein the presence of all three components of said heterodimerizing system allows to form a ternary complex of AB 1/AB2/small molecule,
wherein the affinity between AB 1 and AB2 in the absence of said small molecule is at least 5-fold, at least 20-fold, at least 100-fold, or at least 500-fold weaker (i.e., higher $K_D$ value) compared to the affinity between AB 1 and AB2 in the presence of said small molecule,
and wherein said small molecule is a derivative of xanthine such as caffeine or theophylline.

Said method, wherein said first modification comprises introducing at least one mutation into the area of the interface responsible for the homodimerization of AB0.

Said method, wherein said second modification comprises mutating regions in AB0 that are with regard to their position in spatial proximity to the first modification in AB1, when said ternary complex is built.

Said method, wherein said first modification comprises introducing at least one mutation into the area of the interface responsible for the homodimerization of AB0, and wherein said second modification comprises mutating regions in AB0 that are with regard to their position in spatial proximity to the first modification in AB 1, when said ternary complex is built.

Said method, wherein said first modification comprises introducing at least one mutation into the area of the interface responsible for the homodimerization of AB0 and wherein said second modification comprises mutating regions in AB0 that are with regard to their position in spatial proximity to the first modification in AB1, when said ternary complex is built, as judged from the homodimeric complex structure (PDB-ID 6QTL, wherein one nanobody resembles AB 1 and the other nanobody of this homodimeric structure resembles AB2 of the present invention), wherein the C alpha atom of the second modification in AB2, or at least one of the C alpha atoms of the second modification in AB2 if this modification comprises more than one mutation, is located within 30 Å, within 20 Å, within 15 Å, or within 10 Å from the C alpha atom of any of the mutated position(s) of the first modification in AB 1.

Said second modification that comprises mutating regions in AB0 that may be with regard to their position in spatial proximity to the first modification in AB1, when said ternary complex is built, may have additional mutations in other regions of AB2.

Said method, wherein said first modification may comprise introducing at least one mutation into the area of the interface responsible for the homodimerization of AB0, wherein said interface responsible for the homodimerization of AB0 may be defined as the amino acid positions G29 to V64 and K87 to W109 in SEQ ID NO: 1, determined by analysis of the crystal structure 6QTL, and wherein said second modification may comprise mutating regions in AB0 that are with regard to their position in spatial proximity to the first modification in AB 1, when said ternary complex is built, as judged from the homodimeric complex structure (PDB-ID 6QTL), wherein the C alpha atom of the second modification, or at least one of the C alpha atoms of the second modification if this modification comprises more than one mutation, is located within 30 Å, within 20 Å, within 15 Å, or within 10 Å from the C alpha atom of any of the mutated position(s) of the first modification in AB1.

Said method, wherein said first modification that may comprise introducing at least one mutation into the area of the interface responsible for the homodimerization of AB0 may be an amino acid substitution to a hydrophilic amino acid or an amino acid insertion of a hydrophilic amino acid, wherein said interface responsible for the homodimerization of AB0 may be defined as the amino acid positions G29 to V64 and K87 to W109 in SEQ ID NO: 1, determined by analysis of the crystal structure 6QTL.

Said hydrophilic amino acid may be a positively charged amino acid or a negatively charged amino acid, preferentially a negatively charged amino acid.

Said method, wherein said first modification comprises the amino acid replacement V104D or V104E in SEQ ID NO:1 (AB0), resulting in an antigen binding domain (AB1) having at least 90% or at least 95% identity with the amino acid sequence as set forth in SEQ ID NO:2, if said amino acid replacement is V104D, or having at least 90% or at least 95% identity with the amino acid sequence as set forth in SEQ ID NO:8, if the amino acid replacement is V104E.

Said first modification may be the amino acid substitution V104D or V104E in the amino acid sequence set forth in SEQ ID NO: 1.

Said method, wherein said second modification may comprise mutating regions in AB0 that may be with regard to their position in spatial proximity to the modification in AB 1, when said ternary complex may be built, wherein the C alpha atom of the second modification, or at least one of the C alpha atoms of the second modification if this second modification comprises more than one mutation, is located within 30 Å, within 20 Å, within 15 Å, or within 10 Å from any of the C alpha atoms of any of the mutated position(s) of the first modification in AB1, wherein these distances are based on the C alpha atoms in the structure 6QTL that represents the homodimer of AB0 bound to said small molecule.

Said method, wherein said first modification may be achieved by mutation of at least one amino acid position that is located in close proximity to the second antigen binding domain in the homodimeric complex of ABO/ABO/caffeine (PDB-ID 6QTL).

Said method, wherein said first modification may be achieved by mutation of at least one amino acid that is located in close proximity to the second antigen binding domain in the homodimeric complex of ABO/ABO/caffeine (PDB-ID 6QTL), wherein the C alpha atom of this amino acid is located within 30 Å, within 20 Å, within 15 Å, or within 10 Å from any of the C alpha atoms of the other antigen binding domain in this homodimeric complex of ABO/ABO/caffeine.

Said method, wherein said first modification may be achieved by mutation of at least one amino acid that is located in close proximity to the second antigen binding domain in the homodimeric complex of ABO/ABO/caffeine (PDB-ID 6QTL), wherein the C alpha atom of this amino acid is located within 30 Å, within 20 Å, within 15 Å, or within 10 Å from any of the C alpha atoms of the other antigen binding domain in this homodimeric complex of ABO/ABO/caffeine, and wherein this amino acid is mutated to a hydrophilic amino acid.

Said method, wherein said first modification may be achieved by mutation of at least one amino acid that is located in close proximity to the second antigen binding domain in the homodimeric complex of ABO/ABO/caffeine (PDB-ID 6QTL), wherein the C alpha atom of this amino acid is located within 30 Å, within 20 Å, within 15 Å, or within 10 Å from any of the C alpha atoms of the other antigen binding domain in this homodimeric complex of ABO/ABO/caffeine, and wherein this amino acid is mutated to a negatively charged amino acid.

Said method, wherein said second modification may be achieved by a directed evolution approach.

Methods for directed evolution approaches are well-known in the art. Said directed evolution approach may be based e.g. on a yeast display, a bacterial display, a phage display, a ribosome display or a mammalian cell display approach.

Said yeast display, said bacterial display, said phage display, said ribosome display or said mammalian cell display approach or any other display approach may include a negative selection of the antigen binding domain, i.e., selection or screening for non-binding or binding with reduced affinity to AB 1 in the absence of the small molecule.

Said directed evolution approach that may be based on yeast display may use for selection magnetic beads, flow cytometry or mammalian cell panning.

Said method, wherein preferentially, said small molecule may be caffeine.

Said method, wherein said small molecule may be caffeine, theophylline, theobromine or paraxanthine.

Said method, wherein said small molecule may be caffeine, theophylline, theobromine, paraxanthine, 8-chlorotheophylline, 8-bromotheophylline, doxofylline, dyphylline, pentoxifylline, etofylline, albifylline, proxyphylline, propentofylline, pyridofylline, bamifylline, enprofylline, acefylline, xanthinol, diniprophylline, etamiphylline, lisofylline, pentifylline, arofylline, reproterol, furafylline, dasantafil, IBMX, KMUP-1, KMUP-2, KMUP-3, KMUP-4, theacrine, liberine, methylliberine or 1,3,7-trimethyluric acid.

Said method, wherein said first component and said second component of said chemically-induced heterodimerizing system are nanobodies, respectively.

Said method, wherein said first and said second effector molecules may be proteins, respectively.

Said method, wherein said first and/or second effector molecule may be a DNA binding domain, a transcription activator domain, a transcription repressor domain, an intracellular portion of a receptor, a transmembrane receptor that may comprise one or more extracellular antigen binding domain(s), a transmembrane protein that may comprise one or more intracellular signaling domain(s), a transmembrane receptor that may comprise one or more extracellular antigen binding domain(s) and one or more intracellular signaling domain(s), an intracellular receptor, an extracellular protein, a caspase, a kinase, an enzyme or a protease.

Said method, wherein said AB 1 as a fusion protein and/or said AB2 as a fusion protein may comprise more than one effector molecules, e.g. two or three effector molecules, respectively. Expression of fusion proteins is well known, and fusion proteins can include any appropriate linkers or spacers to facilitate appropriate interaction among various domains.

In another aspect, the present invention provides a (chemically-induced) heterodimerizing system comprising

a) a first component that is a first protein comprising

i) a first antigen binding domain AB1, and
ii) a first effector molecule,

b) a second component that is a second protein comprising

i) a second antigen binding domain AB2, and
ii) a second effector molecule,

c) a third component, wherein said third component is a small molecule,

wherein the presence of all three components of said heterodimerizing system allows to form a ternary complex of AB1/AB2/small molecule,
and wherein said small molecule is a derivative of xanthine such as caffeine or theophylline.

Said heterodimerizing system, wherein said ternary complex has an $EC_{50}$ value of 1 μM or higher, 5 μM or higher, 10 μM or higher, 15 μM or higher, 20 μM or higher, or 50 μM or higher, (as determined by yeast surface display).

Said heterodimerizing system, wherein said AB1 and said AB2 may be derived from a parental antigen binding domain AB0.

Said AB1 and AB2 may be derived from AB0 by the first modification and by the second modification as disclosed herein.

Said heterodimerizing system comprising

a) a first component that is a first protein comprising

i) a first antigen binding domain AB1, and
ii) a first effector molecule,

b) a second component that is a second protein comprising

i) a second antigen binding domain AB2, and
ii) a second effector molecule,

c) a third component, wherein said third component is a small molecule,

wherein the presence of all three components of said heterodimerizing system allows to form a ternary complex of AB1/AB2/small molecule,
and wherein said small molecule is a derivative of xanthine,
wherein said ternary complex of said three components is characterized by a $K_D$ value of 50 μM or below, wherein this $K_D$ defines the affinity between AB 1 and AB2 in the presence of said small molecule, and wherein the binding between AB 1 and the small molecule in the absence of AB2 is either not detectable by SPR or only shows low affinity with a $K_D$ value above 20μM, above 40 μM, above 80 μM, above 160 μM, above 300 μM or above 1 mM (as determined by SPR), if the small molecule is caffeine.

Said heterodimerizing system, wherein said ternary complex of said 3 components may be characterized by a $K_D$ value of 50 μM or below, 5 μM or below, or 500 nM or below, wherein said $K_D$ value defines the affinity between AB 1 and AB2 in the presence of said small molecule. Said heterodimerizing system, wherein the binding between AB 1 and the small molecule in the absence of AB2 may be either not detectable by surface plasmon resonance (SPR) or only may show low affinity with a $K_D$ value above 20 μM, above 40 μM, above 80 μM, above 160 μM, above 300 μM or above 1 mM, respectively.

Said heterodimerizing system, wherein the binding between AB 1 and the small molecule in the absence of AB2 and the binding between AB2 and the small molecule in the absence of AB1 may be either not detectable by surface plasmon resonance (SPR) or only may show low affinity with a $K_D$ value above 20 μM, above 40 μM, above 80 μM, above 160 μM, above 300 μM or above 1 mM, respectively.

Said heterodimerizing system, wherein said ternary complex of said 3 components is characterized by a $K_D$ value of 50 μM or below, 5 μM or below, or 500 nM or below, wherein said $K_D$ value defines the affinity between AB 1 and AB2 in the presence of said small molecule, and wherein the binding between AB 1 and the small molecule in the absence of AB2 may be either not detectable by SPR or only may show low affinity with a $K_D$ value above 20 μM, above 40 μM, above 80 μM, above 160 μM, above 300 μM or above 1 mM, respectively. Said heterodimerizing system, wherein said ternary complex of said 3 components is characterized by a $K_D$ value of 50 μM or below, 5 μM or below, or 500 nM or below, wherein said $K_D$ value defines the affinity between AB 1 and AB2 in the presence of said small molecule, and wherein the binding between AB 1 and the small molecule in the absence of AB2 may be either not detectable by SPR or only may show low affinity with a $K_D$ value above 20 μM, above 40 μM, above 80 μM, above 160 μM, above 300 μM or above 1 mM, respectively, and wherein the affinity between AB1 and AB2 in the absence of the small molecule is at least 5-fold, at least 20-fold, at least 100-fold, or at least 500-fold weaker (i.e., higher $K_D$ value) compared to the affinity between AB 1 and AB2 in the presence of the small molecule.

Said heterodimerizing system, wherein said first antigen binding domain AB1 and said second antigen binding domain AB2 are VHHs or nanobodies.

Said heterodimerizing system, wherein

A) said first antigen binding domain comprises an amino acid sequence having at least 90% or at least 95% identity with SEQ ID NO:2 (acVHH-V104D) with the proviso that amino acid position 104 is not modified,
and wherein said second antigen binding domain comprises an amino acid sequence having at least 75%, at least 80% or at least 85% identity with SEQ ID NO:1 (acVHH-WT) with the proviso that the amino acid position 104 of SEQ ID NO: 1 is not modified, or
B) said first antigen binding domain comprises an amino acid sequence having at least 90% or at least 95% identity with SEQ ID NO:8 (acVHH-V104E) with the proviso that amino acid position 104 is not modified,

and wherein said second antigen binding domain comprises an amino acid sequence having at least 75%, at least 80% or at least 85% identity with SEQ ID NO:1 (acVHH-WT) with the proviso that the amino acid position 104 of SEQ ID NO: 1 is not modified,
and wherein said small molecule is a derivative of xanthine.

Said heterodimerizing system, wherein for said second antigen binding domain of A) or B) the amino acid sequence set forth in SEQ ID NO:1 (acVHH-WT) comprises the modifications:

a) amino acid replacements T50P, V51V, G52A, W53L, S54G, S55L, G56Q and/or I57S,
b) amino acid replacements T50Q, V51Q, G52R, S54L, S55F, G56R and/or I57R,
c) amino acid replacements C22A, S25Y, R27K, T50P, V51V, G52A, W53L, S54G, S55L, G56Q, I57S, C96A, T97A and/or S118T, or
d) amino acid replacements Q1E, C22A, S25Y, T50P, V51V, G52A, W53L, S54G, S55L, G56Q, I57S, C96A and/or T97A.

Said heterodimerizing system, wherein said first antigen binding domain comprises or consists of SEQ ID NO:2 (acVHH-V104D), and wherein said second antigen binding domain comprises or consists of SEQ ID NO:4 (acVHH-M1), SEQ ID NO:5 (acVHH-M2), SEQ ID NO:6 (dfM1-1) or SEQ ID NO:7 (dfM1-2) and wherein said small molecule is a derivative of xanthine.

It was surprisingly found that SEQ ID NO:2 (acVHH-V104D) together with SEQ ID NO:4 (acVHH-M1), SEQ ID NO:6 (dfM1-1) or SEQ ID NO:7 (dfM1-2) works even better in the heterodimerization system as disclosed herein than SEQ ID NO:2 (acVHH-V104D) together with SEQ ID NO:5 (acVHH-M2).

Said heterodimerizing system, wherein preferentially said first antigen binding domain comprises or consists of SEQ ID NO:2 (acVHH-V104D), and wherein said second antigen binding domain comprises or consists of SEQ ID NON (acVHH-M1), SEQ ID NO:6 (dfM1-1) or SEQ ID NO:7 (dfM1-2), and wherein said small molecule is a derivative of xanthine.

It was surprisingly found that SEQ ID NO:2 (acVHH-V104D) together with SEQ ID NO:6 (dfM1-1) or SEQ ID NO:7 (dfM1-2) works even better in the heterodimerization system as disclosed herein, especially when expressed intracellularly in a cell, than SEQ ID NO:2 (acVHH-V104D) together with SEQ ID NO:4 (acVHH-M1).

Said heterodimerizing system, wherein more preferentially said first antigen binding domain comprises or consists of SEQ ID NO:2 (acVHH-V104D), and wherein said second antigen binding domain comprises or consists SEQ ID NO:6 (dfM1-1) or SEQ ID NO:7 (dfM1-2), and wherein said small molecule is a derivative of xanthine.

Said heterodimerizing system as disclosed herein, wherein said first antigen binding domain comprises one or more amino acid modifications in the amino acid sequence set forth in SEQ ID NO:1 (acVHH-WT) with the proviso that one amino acid modification is the amino acid replacement V104D or V104E, and wherein said modifications in the amino acid sequence of the first antigen binding domain are not more than 15, 14, 13, 12, 11, 10, 9, 8, 7, 6, 5, 4, 3, 2 or 1 modifications, wherein said modifications of said first antigen binding domain are selected from an amino acid replacement, an amino acid deletion or an amino acid insertion,

and wherein said second antigen binding domain comprises six or more amino acid modifications in the amino acid sequence set forth in SEQ ID NO:1 (acVHH-WT) with the proviso that the amino acid replacement V104D or V104E is not one of said modifications in the second antigen binding domain,
and wherein said modifications in the amino acid sequence of the second antigen binding domain are not more than 25, 24, 23, 22, 21, 20, 19, 18, 17, 16, 15 or 14 modifications, wherein said modifications of said second antigen binding domain are selected from an amino acid replacement, an amino acid deletion or an amino acid insertion,
and wherein said small molecule is a derivative of xanthine.

Said heterodimerizing system, wherein for said second antigen binding domain said six or more amino acid modifications in the amino acid sequence set forth in SEQ ID NO:1 (acVHH-WT) comprise

a) amino acid replacements T50P, V51V, G52A, W53L, S54G, S55L, G56Q and/or I57S,
b) amino acid replacements T50Q, V51Q, G52R, S54L, S55F, G56R and/or I57R,
c) amino acid replacements C22A, S25Y, R27K, T50P, V51V, G52A, W53L, S54G, S55L, G56Q, I57S, C96A, T97A and/or S118T, or
d) amino acid replacements Q1E, C22A, S25Y, T50P, V51V, G52A, W53L, S54G, S55L, G56Q, I57S, C96A and/or T97A.

Said heterodimerizing system, wherein said small molecule is caffeine.

Said heterodimerizing system, wherein said first and/or second effector molecule is an intracellular portion of a receptor, a transmembrane receptor that may comprise one or several extracellular antigen binding domain(s), a transmembrane protein that may comprise one or several intracellular signaling domain(s), a transmembrane receptor that may comprise one or several extracellular antigen binding domain(s) and one or several intracellular signaling domain(s), an intracellular receptor, an extracellular protein, a caspase, a kinase, an enzyme or a protease.

In another aspect, the present invention provides a (chemically-induced) heterodimerizing system, wherein said system may be obtained by the method as disclosed herein.

In another aspect, the present invention provides a (chemically-induced) heterodimerizing system comprising

a) a first component that is a first protein comprising

i) a first antigen binding domain AB1 comprising SEQ ID NO:2 (acVHH-V104D), and
ii) a first effector molecule,

b) a second component that is a second protein comprising

i) a second antigen binding domain AB2 comprising SEQ ID NO:4 (acVHH-M1), SEQ ID NO:5 (acVHH-M2), SEQ ID NO:6 (dfM1-1) or SEQ ID NO:7 (dfM1-2), and
ii) a second effector molecule,

c) a third component, wherein said third component is a small molecule,
and wherein said small molecule is a derivative of xanthine such as caffeine or theophylline.

In another aspect, the present invention provides a composition comprising

a) a first component that is a first protein comprising

i) a first antigen binding domain AB1, and
ii) a first effector molecule,

b) a second component that is a second protein comprising

i) a second antigen binding domain AB2, and
ii) a second effector molecule,

c) a third component, wherein said third component is a small molecule,

wherein the presence of all three components allows to form a ternary complex of AB1/AB2/small molecule, and wherein said small molecule is a derivative of xanthine such as caffeine or theophylline.

In another aspect, the present invention provides a cell comprising (or expressing)

a) a first component that is a first protein comprising

i) a first antigen binding domain AB1, and
ii) a first effector molecule,

b) a second component that is a second protein comprising

i) a second antigen binding domain AB2, and
ii) a second effector molecule,
wherein the presence of a third component that is a small molecule allows to form a ternary complex, wherein said ternary complex is a ternary complex of AB 1/AB2/small molecule, and wherein said small molecule is a derivative of xanthine such as caffeine or theophylline.

Said cell may be an immune cell such as a T cell, a B cell, or an NK cell.
Said immune cell may be a cell expressing a transgene, wherein said transgene may comprise said first component and said second component as disclosed herein.
Said immune cell may be a cell expressing a CAR, wherein said CAR may comprise said first component and said second component as disclosed herein.
Said cell, wherein said first effector molecule of said first protein may comprise

a) an antigen binding domain specific for an antigen expressed on the surface of a target cell or specific for a soluble antigen
b) a transmembrane domain
c) an endodomain optionally comprising an intracellular signaling domain,

and wherein said endodomain of said first effector molecule may comprise said first antigen binding domain AB1, and wherein said second effector molecule of said second protein may comprise

a) a transmembrane domain
b) an endodomain comprising an intracellular signaling domain,
and wherein said endodomain of said second effector molecule may comprise said second antigen binding domain AB2.

Said cell, wherein said first effector molecule of said first protein may comprise

a) a transmembrane domain
b) an endodomain comprising an intracellular signaling domain,

and wherein said endodomain of said first effector molecule may comprise said first antigen binding domain AB1, and wherein said second effector molecule of said second protein may comprise

a) an antigen binding domain specific for an antigen expressed on the surface of a target cell or specific for a soluble antigen
b) a transmembrane domain
c) an endodomain optionally comprising an intracellular signaling domain,
and wherein said endodomain of said second effector molecule may comprise said second antigen binding domain AB2.

Said intracellular signaling domain of said first effector molecule may comprise or may consist of a co-stimulatory domain, and said intracellular signaling domain of said second effector molecule may comprise or may consist of a stimulatory domain.

In one embodiment of the invention a cell comprises

a) a first component that is a first protein comprising

i) a first antigen binding domain AB 1, and
ii) a first effector molecule,

b) a second component that is a second protein comprising

i) a second antigen binding domain AB2, and
ii) a second effector molecule,
wherein the presence of a third component that is a small molecule allows to form a ternary complex, wherein said ternary complex is a ternary complex of AB 1/AB2/small molecule, and wherein said small molecule is a derivative of xanthine such as caffeine or theophylline, and wherein said first effector molecule of said first protein may comprise

a) an antigen binding domain specific for an antigen expressed on the surface of a target cell or specific for a soluble antigen
b) a transmembrane domain
c) an endodomain optionally comprising an intracellular signaling domain comprising or consisting of a co-stimulatory domain,

and wherein said endodomain of said first effector molecule may comprise said first antigen binding domain AB1,
and wherein said second effector molecule of said second protein may comprise

a) a transmembrane domain
b) an endodomain comprising an intracellular signaling domain comprising a stimulatory signaling domain and optionally a co-stimulatory domain,
and wherein said endodomain of said second effector molecule may comprise said second antigen binding domain AB2.

Said cell, wherein said first effector molecule of said first protein may comprise

a) a first antigen binding domain specific for a first antigen expressed on the surface of a target cell or specific for a first soluble antigen
b) a transmembrane domain
c) an endodomain optionally comprising an intracellular signaling domain comprising a co-stimulatory domain and/or a stimulatory domain,

and wherein said endodomain of said first effector molecule may comprise said first antigen binding domain AB1,
and wherein said second effector molecule of said second protein may comprise

a) a second antigen binding domain specific for a second antigen expressed on the surface of a target cell or specific for a second soluble antigen
b) a transmembrane domain
c) an endodomain optionally comprising an intracellular signaling domain comprising a co-stimulatory domain and/or a stimulatory domain,

and wherein said endodomain of said second effector molecule may comprise said second antigen binding domain AB2.

In another aspect, the present invention provides a nucleic acid sequence or two or more nucleic acid sequences encoding for

a) a first component that is a first protein comprising

i) a first antigen binding domain AB1, and
ii) a first effector molecule,

b) a second component that is a second protein comprising

i) a second antigen binding domain AB2, and
ii) a second effector molecule,

wherein the presence of a third component that is a small molecule allows to form a ternary complex of AB 1/AB2/small molecule,
and wherein said small molecule is a derivative of xanthine such as caffeine or theophylline.

In another aspect, the present invention provides an in-vivo method for treating a disease such as cancer, an autoimmune disease or an infectious disease in a subject comprising

A) administering to said subject a genetically engineered immune cell comprising

a) a first component that is a first protein comprising

i) a first antigen binding domain AB1, and
ii) a first effector molecule,

b) a second component that is a second protein comprising

i) a second antigen binding domain AB2, and
ii) a second effector molecule,

B) administering to said subject a small molecule,

wherein the presence of said small molecule as a third component allows to form a ternary complex within or on said immune cell, wherein said ternary complex is a ternary complex of AB1/AB2/small molecule,
and wherein said small molecule is a derivative of xanthine such as caffeine or theophylline.

Said immune cell may be a T cell, a B cell, or an NK cell.
All definitions, characteristics and embodiments defined herein with regard to an aspect of the invention, e.g. the first aspect of the invention, also apply mutatis mutandis in the context of the other aspects of the invention as disclosed herein.

Embodiments

Effector molecules may be e.g. those that regulate a process in a cell when dimerized. Thus, effector molecules may be e.g. be portions of receptors (e.g., intracellular receptors), various components of CAR molecules, caspases, enzymes, kinases, extracellular proteins etc. Prominent examples may be the use in CARs as disclosed herein in more detail. However, it is not to be intended to limit the types of effector molecules to these illustrative embodiments.
In one embodiment of the invention, the chemically-induced heterodimerizing system is a chimeric antigen receptor (CAR) regulatable system comprising

a) a first polypeptide comprising

i) an antigen binding domain specific for an antigen, e.g. specific for an antigen expressed on a target cell such as a tumor cell (e.g., a tumor-associated antigen (TAA))
ii) a transmembrane domain
iii) a first dimerizer binding domain (the antigen binding domain AB 1 as disclosed herein) comprising e.g. SEQ ID NO:2 (acVHH-V104D), and optionally
iv) an intracellular signaling domain comprising a co-stimulatory domain,

b) a second polypeptide comprising

i) a transmembrane domain
ii) a second dimerizer binding domain comprising (the antigen binding domain AB2 as disclosed herein) e.g. SEQ ID NO:4 (acVHH-M1), SEQ ID NO:5 (acVHH-M2), SEQ ID NO:6 (dfM1-1) or SEQ ID NO:7 (dfM1-2)
iii) an intracellular signaling domain comprising a co-stimulatory domain and/or a stimulatory domain

c) a small molecule (the dimerizer), wherein said small molecule is a derivative of xanthine such as caffeine or theophylline,
wherein the presence of the first polypeptide, the second polypeptide and the small molecule allows the heterodimerization of the two CAR components, i.e. the first polypeptide and the second polypeptide via said small molecule.

In another embodiment of the invention, the first dimerizer binding domain of the preceding embodiment may comprise SEQ ID NO:4 (acVHH-M1), SEQ ID NO:5 (acVHH-M2), SEQ ID NO:6 (dfM1-1) or SEQ ID NO:7 (dfM1-2) instead of SEQ ID NO:2 (acVHH-V104D) and the second dimerizer binding domain of the preceding embodiment may comprise SEQ ID NO:2 (acVHH-V104D) instead of SEQ ID NO:4 (acVHH-M1), SEQ ID NO:5 (acVHH-M2), SEQ ID NO:6 (dfM1-1) or SEQ ID NO:7 (dfM1-2).

In one embodiment of the invention, an immune cell expresses a regulatable chimeric antigen receptor (CAR) comprising

a) a first polypeptide comprising

i) an antigen binding domain specific for an antigen, e.g. specific for an antigen expressed on a target cell such as a tumor cell (e.g., a tumor-associated antigen (TAA))
ii) a transmembrane domain
iii) a first dimerizer binding domain comprising (the antigen binding domain AB1 as disclosed herein) e.g. SEQ ID NO:2 (acVHH-V104D), and optionally
iv) an intracellular signaling domain comprising a co-stimulatory domain,

b) a second polypeptide comprising

i) a transmembrane domain
ii) a second dimerizer binding domain comprising (the antigen binding domain AB2 as disclosed herein) e.g. SEQ ID NO:4 (acVHH-M1), SEQ ID NO:5 (acVHH-M2), SEQ ID NO:6 (dfM1-1) or SEQ ID NO:7(dfM1-2)
iii) an intracellular signaling domain comprising a co-stimulatory domain and/or a stimulatory domain
wherein in the presence of a small molecule (a dimerizer) the first polypeptide and the second polypeptide heterodimerize and are able to form a ternary complex via the small molecule within said immune cell, and wherein said small molecule is a derivative of xanthine such as caffeine or theophylline.

In another embodiment of the invention, the first dimerizer binding domain of the preceding embodiment may comprise SEQ ID NO:4 (acVHH-M1), SEQ ID NO:5 (acVHH-M2), SEQ ID NO:6 (dfM1-1) or SEQ ID NO:7 (dfM1-2) instead of SEQ ID NO:2 (acVHH-V104D) and the second dimerizer binding domain of the preceding embodiment may comprise SEQ ID NO:2 (acVHH-V104D) instead of SEQ ID NO:4 (acVHH-M1), SEQ ID NO:5 (acVHH-M2), SEQ ID NO:6 (dfM1-1) or SEQ ID NO:7 (dfM1-2).

A special CAR platform with inducible and logic control functions is the avidity-controlled CAR (AvidCAR) which is disclosed in Salzer et al. (NATURE COMMUNICATIONS, 2020: 11:4166; WO2020070289A1). The key design principles of an AvidCAR were (1) the development of strictly monomeric CAR backbones by removing cysteines in the hinge region to prevent uncontrolled disulfide-mediated CAR dimerization; (2) the incorporation of antigen-binding domains whose affinities are substantially reduced down to levels comparable to those of TCRs ($K_D$ > 0.1-1000 $\mu$M); and (3) the optional use of single-domain antigen-binding domains, which seems to be preferable to exclude potential oligomerization that has been observed for some scFvs.

Therefore, in one embodiment of the present invention the chemically-induced heterodimerizing system is an AvidCAR system comprising

a) a first polypeptide comprising

i) a first antigen binding domain specific for an antigen, e.g. specific for an antigen expressed on a target cell such as a tumor cell (e.g., a tumor-associated antigen (TAA)), and wherein the affinity of said first antigen binding domain to said antigen may be between 1 mM and 100 nM
ii) a hinge region (or spacer)
iii) a transmembrane domain
iv) a first dimerizer binding domain (the antigen binding domain AB1 as disclosed herein) comprising e.g. SEQ ID NO:2 (acVHH-V104D), and optionally
v) an intracellular signaling domain comprising a stimulatory domain and/or co-stimulatory domain

b) a second polypeptide comprising

i) a second antigen binding domain specific for an antigen, e.g. specific for an antigen expressed on a target cell such as a tumor cell (e.g., a tumor-associated antigen (TAA)), and wherein the affinity of said second antigen binding domain to said antigen may be between 1 mM and 100 nM
ii) a hinge region (or spacer)
iii) a transmembrane domain
iv) a second dimerizer binding domain (the antigen binding domain AB2 as disclosed herein) comprising e.g. SEQ ID NO:4 (acVHH-M1), SEQ ID NO:5 (acVHH-M2), SEQ ID NO:6 (dfM1-1) or SEQ ID NO:7 (dfM1-2)
v) an intracellular signaling domain comprising a stimulatory domain and/or a co-stimulatory domain

c) a small molecule (the dimerizer), wherein said small molecule is a derivative of xanthine such as caffeine or theophylline,

wherein the extracellular domains of both polypeptides in their prevalent (native) conformations are free of cysteine amino acid moieties which are able to form intermolecular disulphide bonds with the other polypeptide, respectively, or with another copy of the same polypeptide,
and wherein only the presence of the first polypeptide, the second polypeptide and the small molecule allows the heterodimerization of the two CAR components, i.e. the first polypeptide and the second polypeptide via said small molecule.

Said first antigen binding domain and said second antigen binding domain may be identical or may be different antigen binding domains that may bind to different antigens.

In another embodiment of the invention, the first dimerizer binding domain of the preceding embodiment may comprise SEQ ID NO:4 (acVHH-M1), SEQ ID NO:5 (acVHH-M2), SEQ ID NO:6 (dfM1-1) or SEQ ID NO:7 (dfM1-2) instead of SEQ ID NO:2 (acVHH-V104D) and the second dimerizer binding domain of the preceding embodiment may comprise SEQ ID NO:2 (acVHH-V104D) instead of SEQ ID NO:4 (acVHH-M1), SEQ ID NO:5 (acVHH-M2), SEQ ID NO:6 (dfM1-1) or SEQ ID NO:7 (dfM1-2).

In one embodiment of the invention, an immune cell expresses an AvidCAR comprising

a) a first polypeptide comprising

i) a first antigen binding domain specific for an antigen, e.g. specific for an antigen expressed on a target cell such as a tumor cell (e.g., a tumor-associated antigen (TAA)), and wherein the affinity of said first antigen binding domain to said antigen may be between 1 mM and 100nM
ii) a hinge region (or spacer)
iii) a transmembrane domain
iv) a first dimerizer binding domain (the antigen binding domain AB 1 as disclosed herein) comprising e.g. SEQ ID NO:2 (acVHH-V104D), and optionally
v) an intracellular signaling domain comprising a stimulatory domain and/or co-stimulatory domain

b) a second polypeptide comprising

i) a second antigen binding domain specific for an antigen, e.g. specific for an antigen expressed on a target cell such as a tumor cell (e.g., a tumor-associated antigen (TAA)), and wherein the affinity of said second antigen binding domain to said antigen may be between 1 mM and 100nM
ii) a hinge region (or spacer)
iii) a transmembrane domain
iv) a second dimerizer binding domain (the antigen binding domain AB2 as disclosed herein) comprising e.g. SEQ ID NO:4 (acVHH-M1), SEQ ID NO:5 (acVHH-M2), SEQ ID NO:6 (dfM1-1) or SEQ ID NO:7 (dfM1-2)
v) an intracellular signaling domain comprising a stimulatory domain and/or a co-stimulatory domain

wherein the extracellular domains of both polypeptides in their prevalent (native) conformations are free of cysteine amino acid moieties which are able to form intermolecular disulphide bonds with the other polypeptide, respectively, or with another copy of the same polypeptide,
and wherein in the presence of a small molecule (a dimerizer) the first polypeptide and the second polypeptide heterodimerize and are able to form a ternary complex via the small molecule within said immune cell, and wherein said small molecule is a derivative of xanthine such as caffeine or theophylline.

In another embodiment of the invention, the first dimerizer binding domain of the preceding embodiment may comprise SEQ ID NO:4 (acVHH-M1), SEQ ID NO:5 (acVHH-M2), SEQ ID NO:6 (dfM1-1) or SEQ ID NO:7 (dfM1-2) instead of SEQ ID NO:2 (acVHH-V104D) and the second dimerizer binding domain of the preceding embodiment may comprise SEQ ID NO:2 (acVHH-V104D) instead of SEQ ID NO:4 (acVHH-M1), SEQ ID NO:5 (acVHH-M2), SEQ ID NO:6 (dfM1-1) or SEQ ID NO:7 (dfM1-2).

In one embodiment of the invention, immune cells expressing an AvidCAR as disclosed herein or a split CAR as disclosed herein or a CAR that may be regulatable by a transcription factor as disclosed herein are for use in treatment of a disease associated with a target cell of a subject suffering from said disease, the disease may be e.g. cancer, an autoimmune disease or an infectious disease and the target cell may be e.g. a cancerous cell. Immune cells, e.g. T cells or NK cells of a subject may be isolated. The subject may e.g. suffer from said cancer or may be a healthy subject. These cells may be genetically modified *in vitro* to express the AvidCAR or split CAR as disclosed herein. These engineered cells may be activated and expanded *in vitro* or *in vivo* to therapeutic effective amounts using methods known in the art. In a cellular therapy, these engineered cells may be infused to a recipient in need thereof. These cells may be a pharmaceutical composition (said cell plus pharmaceutical acceptable carrier). The infused cells may be e.g. able to kill (or at least stop growth of) cancerous cells in the recipient. The recipient may be the same subject from which the cells were obtained (autologous cell therapy) or may be another subject of the same species (allogenic cell therapy).

The immune cells, preferentially T cells or NK cells engineered to express the CARs as described above and disclosed herein may be administered either alone, or as a pharmaceutical composition in combination with diluents and/or with other components such as IL-2 or other cytokines or cell populations. Briefly, pharmaceutical compositions of the present invention may comprise a cell population of genetically modified cells (a plurality of immune cells) as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives.

Preferentially, the compositions of the present invention are formulated for intravenous administration. The administration of cell compositions to the subject may be carried out in any convenient manner known in the art.

Pharmaceutical compositions of the present invention may be administered in a manner appropriate to the disease to be treated. Appropriate dosages may be determined by clinical trials. However, the quantity and frequency of administration will also be determined and influenced by such factors as the condition of the patient, and the type and severity of the patient's disease.

A pharmaceutical composition comprising the immune cells, preferentially T cells or NK cells as disclosed herein may be administered at a dosage of $10^4$ to $10^9$ cells/kg body weight, preferably $10^5$ to $10^6$ cells/kg body weight. The cell compositions may also be administered several times at these dosages. The compositions of cells may be injected e.g. directly into a tumor, lymph node, or site of infection.

In addition to the pharmaceutical composition comprising said genetically engineered immune cells, the dimerizer (i.e. the small molecule) being a derivative of xanthine such as caffeine or theophylline is administered to said subject intended to be treated, said administration may be orally, e.g. said subject may consume caffeine-containing foods or beverages such as coffee, tea and/or chocolate. Said dimerizer (i.e. the small molecule) allows the generation of the complete and functional CARs as mentioned above in said immune cells. Said dimerizer (i.e., the small molecule) may be administered, e.g. orally administered to said subject simultaneously with the administration of said immune cells, or may be administered/consumed subsequently, e.g. one day or one week later, either once or repeatedly. Moreover, since it has been shown that transient rest restores T cell functionality and prevents exhaustion (Weber, E.W. et al., 2021, Science, 372(6537):eaba1786), administration and/or consumption of the dimerizer (i.e., the small molecule) may optionally alternate with transient pauses.

Definitions

Unless defined otherwise, technical and scientific terms used herein have the same meaning as commonly understood by one of ordinary skill in the art to which this invention belongs. As used herein the term "comprising" or "comprises" is used in reference to compositions, methods, and respective component(s) thereof, that are essential to the method or composition, yet open to the inclusion of unspecified elements, whether essential or not.

The term "derivative of xanthine" refers to derivatives of the compound xanthine and are a group of alkaloids, some of which are known for their effects as mild stimulants.

The compound xanthine is a purine base found in most human body tissues and fluids, as well as in other organisms. Several compounds (the derivatives of xanthine) are derived from xanthine, including caffeine, theophylline, theobromine, paraxanthine, 8-chlorotheophylline, 8-bromotheophylline, doxofylline, dyphylline, pentoxifylline, etofylline, albifylline, proxyphylline, propentofylline, pyridofylline, bamifylline, enprofylline, acefylline, xanthinol, diniprophylline, etamiphylline, IBMX, KMUP-1, KMUP-2, KMUP-3, KMUP-4, theacrine, liberine, methylliberine and 1,3,7-trimethyluric acid. Herein most preferred derivatives of xanthine are caffeine, theophylline and theobromine.

The term "chemically-induced dimerizing system" or "chemically-induced protein dimerizing system" refers to a system wherein two proteins which do not normally interact, pair in the presence of a small molecule (a dimerizer), or wherein the affinity between these two proteins is enhanced in the presence of the small molecule. In this context, a homodimerization means that the interaction of two identical proteins is enhanced by the small molecule, a heterodimerization means that the interaction of two different proteins is enhanced by the small molecule.

The term "protein complementation assay" (or also termed NanoBiT® complementation assay) defines an assay system for analysis of protein:protein interaction based on the "NanoBiT® Protein:Protein Interaction System" marketed by Promega and previously described by Dixon et al. (ACS Chem Biol. 2016 Feb 19;11(2):400-8.). This system uses a split NanoLuc® luciferase in which the two subunits Large BiT (LgBiT) and Small BiT (SmBiT) are fused with two desired proteins of interest. When the two proteins of interest interact with each other, the two subunits form a functional NanoLuc® luciferase, which produces a luminescent signal in the presence of substrate.

Surface plasmon resonance (SPR) is the resonant oscillation of conduction electrons at the interface between negative and positive permittivity material stimulated by incident light. SPR is the basis of many standard tools for measuring adsorption of material onto planar metal (typically gold or silver) surfaces. SPR is considered a standard technique to measure affinities between biomolecules.

The term affinity ($K_D$) defines the equilibrium dissociation constant describing the interaction between two molecules. In general, the $K_D$ value corresponds to the ligand concentration that is required to yield 50% saturation of its interaction partner. In the present invention, in the case of the interaction between AB1 and AB2 in the absence of the small molecule, the term affinity ($K_D$) is used to describe the 1: 1 interaction between AB1 and AB2, i.e. the concentration of soluble AB1 or AB2 or of soluble fusion proteins containing AB1 or AB2 that is required to yield 50% complexation of the interaction partner, respectively. In the present invention, in the case of the interaction between AB1 and AB2 in the presence of the small molecule (at a concentration that is within 20-fold below or above the $EC_{50,YSD}$ (determined in yeast display experiments) of that small molecule, i.e. the concentration is between 20-fold below the $EC_{50}$ and 20-fold above the $EC_{50}$), the affinity ($K_D$) is similarly used to describe the ligand concentration (e.g., soluble AB1 or AB2 or soluble fusion proteins containing AB1 or AB2) that is required to achieve 50% of the binding signal. Thus, even though these systems represent complexes consisting of three components (i.e., not a classical 1:1 interaction), the term affinity ($K_D$) is used to describe the AB1-AB2 interaction in the presence of the small molecule in order to facilitate comparison with the affinity ($K_D$) in the absence of the small molecule. Furthermore, the affinity ($K_D$) is also used to describe the interaction between AB1 and the small molecule in the absence of AB2. This affinity between AB1 and the small molecule is preferentially determined by SPR.

In the present invention, the $EC_{50,YSD}$ of the small molecule is defined as the concentration of the small molecule that is required to yield 50% complexation of a yeast displayed antigen binding domain (AB1 or AB2) with its soluble interaction partner (the other antigen binding domain, i.e., AB2 or AB1, respectively, or fusion proteins containing AB2 or AB1, respectively), wherein the concentration of the soluble interaction partner is 10-50-fold above its affinity ($K_{D,}$ in this case referring to the affinity between AB1 and AB2) determined in the presence of the small molecule as described above.

In the present invention, the $EC_{50,NanoBiT®}$ of the small molecule is defined as the concentration of the small molecule that is required to yield 50% of the maximum signal in a NanoBiT® assay (protein complementation assay).

The term "$EC_{50}$" as used herein normally refers to $EC_{50}$ values determined in yeast display experiments (=$EC_{50,YSD}$). If the $EC_{50}$ values are determined in a NanoBiT® assay as described herein, then it is explicitly termed as "$EC_{50,NanoBiT®}$".

As used herein, the term "identity" (of proteins and polypeptides) with respect to amino acid sequences is used for a comparison of protein chains. Calculations of "sequence identity" between two sequences are performed as follows. The sequences are aligned for optimal comparison purposes (e.g., gaps can be introduced in one or both of a first and a second amino acid sequence for optimal alignment and non-homologous sequences can be disregarded for comparison purposes). The optimal alignment is determined as the best score using the "ssearch36" program in the FASTA36 software package (http://faculty.virginia.edu/wrpearson/fasta/) with a Blossum 50 scoring matrix with a gap-open penalty of -10, and a gap-extension penalty of -2. The amino acid residues at corresponding amino acid positions are then compared. When a position in the first sequence is occupied by the same amino acid residue at the corresponding position in the second sequence, then the molecules are identical at that position. The percent identity between the two sequences is a function of the number of identical positions shared by the sequences.

The term "yeast surface display" or "YSD" or "yeast display" as used herein describes a system, where Aga2p-based fusion proteins (optionally also including linkers and/or expression tags such as a c-myc-tag and/or an HA-tag) are expressed on the surface of *S. cerevisiae* (strain EBY100). The terms "yeast surface display" or "YSD" or "yeast display" as used herein may be used interchangeably. Yeast surface display based on Aga2p-anchoring on the yeast is well-described in the field (Chao et al., Nat Protoc. 2006;1(2):755-768; Angelini et al., Methods Mol Biol. 2015;1319:3-36; Chen et al., Methods Enzymol. 2013;523:303-26) and well-known to a person skilled in the art.

In general, a CAR as used herein may comprise an extracellular domain (extracellular part) comprising the antigen binding domain, a transmembrane domain and a cytoplasmic signaling domain (intracellular signaling domain). The extracellular domain may be linked to the transmembrane domain by a linker or spacer. The extracellular domain may also comprise a signal peptide. In some embodiments of the invention the antigen binding domain of a CAR binds a tag or hapten that is coupled to a polypeptide ("haptenylated" or "tagged" polypeptide), wherein the polypeptide may bind to a disease-associated antigen such as a tumor associated antigen (TAA) that may be expressed on the surface of a cancer cell. Such a CAR may be referred to as "anti-tag" CAR or "adapterCAR" or "universal CAR" as disclosed e.g. in US9233125B2.

The haptens or tags may be coupled directly or indirectly to a polypeptide (the tagged polypeptide), wherein the polypeptide may bind to said disease associated antigen expressed on the (cell) surface of a target. The tag may be e.g. dextran or a hapten such as biotin or fluorescein isothiocyanate (FITC) or phycoerythrin (PE) or thiamin, but the tag may also be a peptide sequence e.g. chemically or recombinantly coupled to the polypeptide part of the tagged polypeptide. The tag may also be streptavidin. The tag portion of the tagged polypeptide is only constrained by being a molecular component that can be recognized and specifically bound by the antigen binding domain specific for the tag of the CAR. For example, when the tag is FITC (fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv, respectively.

A "signal peptide" refers to a peptide sequence that directs the transport and localization of the protein within a cell, e.g. to a certain cell organelle (such as the endoplasmic reticulum) and/or to the cell surface.

Generally, an "antigen binding domain" in the context of a CAR (not to be confused with the antigen binding domains AB0, AB1 and AB2 as disclosed herein) refers to the region of the CAR that specifically binds to an antigen, e.g. to a tumor associated antigen (TAA) or tumor specific antigen (TSA). The CARs of the invention may comprise one or more antigen binding domains (e.g. a tandem CAR). Generally, the targeting regions on the CAR are extracellular. The antigen binding domain may comprise an antibody or an antigen binding fragment thereof. The antigen binding domain may comprise, for example, full length heavy chain, Fab fragments, single chain Fv (scFv) fragments, divalent single chain antibodies, diabodies or single domain antibodies (VHHs or nanobodies). Any molecule that binds specifically to a given antigen such as affibodies, DARPins, Sso7d-based binders, Sac7d-based binders, monobodies, or any other engineered binding scaffold (Zajc, C.U. et al., 2021, FEBS J. 288(7):2103-2118) or ligand binding domains from naturally occurring receptors or ligands may be used as an antigen binding domain. Often the antigen binding domain is a scFv or single domain antibody. Normally, in a scFv, the variable regions of an immunoglobulin heavy chain and light chain are fused by a flexible linker to form a scFv. Such a linker may be for example the "$(G_4/S)_3$-linker".

In some instances, it is beneficial for the antigen binding domain to be derived from the same species in which the CAR will be used in. For example, when it is planned to use it therapeutically in humans, it may be beneficial for the antigen binding domain of the CAR to comprise a human or humanized antibody or antigen binding fragment thereof or binders based on other human protein domains such as monobodies that are based on the human FN3 domain. Human or humanized antibodies or antigen binding fragments thereof or engineered binders based on other human protein domains can be made by a variety of methods well known in the art.

"Spacer" or "hinge" as used herein refers to the hydrophilic region which is between the antigen binding domain and the transmembrane domain. The CARs of the invention may comprise an extracellular spacer domain but it is also possible to leave out such a spacer. The spacer may include e.g. Fc fragments of antibodies or fragments thereof, hinge regions of antibodies or fragments thereof, CH2 or CH3 regions of antibodies, accessory proteins, artificial spacer sequences or combinations thereof. A prominent example of a spacer is the CD8alpha hinge.

The transmembrane domain of the CAR may be derived from any desired natural or synthetic source for such domain. When the source is natural the domain may be derived from any membrane-bound or transmembrane protein. The transmembrane domain may be derived for example from CD8alpha or CD28. When the key signaling and antigen recognition modules (domains) are on two (or even more) polypeptides then the CAR may have two (or more) transmembrane domains. Separating the key signaling and antigen recognition modules on two different polypeptide chains enables small molecule-dependent, titratable and reversible control over CAR signaling (e.g. WO2014127261A1) due to small molecule-dependent heterodimerizing domains in each polypeptide of the CAR, i.e. by using the antigen binding domains AB 1 and AB2, respectively, which are disclosed in the present invention.

The cytoplasmic signaling domain (the intracellular signaling domain or the activating endodomain) of the CAR is responsible for activation of at least one of the normal effector functions of the immune cell in which the CAR is expressed, if the respective CAR is an activating CAR (normally, a CAR as described herein refers to an activating CAR, otherwise it is indicated explicitly as an inhibitory CAR (iCAR)). "Effector function" means a specialized function of a cell, e.g. in a T cell an effector function may be cytolytic activity or helper activity including the secretion of cytokines. The intracellular signaling domain refers to the part of a protein which transduces the effector function signal and directs the cell expressing the CAR to perform a specialized function. The intracellular signaling domain may include any complete, mutated or truncated part of the intracellular signaling domain of a given protein sufficient to transduce a signal which initiates or blocks immune cell effector functions.

Prominent examples of intracellular signaling domains for use in the CARs include the cytoplasmic signaling sequences of the T cell receptor (TCR) complex, co-receptors and co-stimulatory receptors that initiate signal transduction following receptor engagement.

Generally, T cell activation can be mediated by two distinct classes of cytoplasmic signaling sequences, firstly those that initiate antigen-dependent primary activation through the TCR complex (primary cytoplasmic signaling sequences, primary cytoplasmic signaling domain) and secondly those that usually act in an antigen-independent manner to provide a secondary or co- stimulatory signal (secondary cytoplasmic signaling sequences, co-stimulatory signaling domain). Therefore, an intracellular signaling domain of a CAR may comprise one or more primary cytoplasmic signaling domains and/or one or more secondary cytoplasmic signaling domains.

Primary cytoplasmic signaling domains that act in a stimulatory manner may contain ITAMs (immunoreceptor tyrosine-based activation motifs).

Examples of ITAM containing primary cytoplasmic signaling domains often used in CARs are those derived from TCRζ (CD3ζ), FcRgamma, FcRbeta, CD3gamma, CD3delta, CD3epsilon, CD5, CD22, CD79a, CD79b, and CD66d. Most prominent is sequence derived from CD3ζ.

The cytoplasmic domain of the CAR may be designed to comprise the CD3ζ signaling domain by itself or combined with any other desired cytoplasmic domain(s). The cytoplasmic domain of the CAR can comprise a CD3ζ chain portion and a co-stimulatory signaling region (domain). The co-stimulatory signaling region refers to a part of the CAR comprising the intracellular domain of a co-stimulatory molecule. A co-stimulatory molecule is a cell surface molecule other than an antigen receptor or their ligands that is required for an efficient response of lymphocytes to an antigen. Examples for a co-stimulatory molecule are CD27, CD28, 4-1BB (CD137), OX40, CD30, CD40, ICOS, lymphocyte function-associated antigen- 1 (LFA-1), CD2, CD7, LIGHT, NKG2C, B7-H3.

The cytoplasmic signaling sequences within the cytoplasmic signaling part of the CAR may be linked to each other with or without a linker in a random or specified order. A short oligo- or polypeptide linker, which is preferably between 2 and 10 amino acids in length, may form the linkage. A prominent linker is the glycine-serine doublet.

As an example, the cytoplasmic domain may comprise the signaling domain of CD3ζ and the signaling domain of CD28. In another example, the cytoplasmic domain may comprise the signaling domain of CD3ζ and the signaling domain of CD137. In a further example, the cytoplasmic domain may comprise the signaling domain of CD3ζ, the signaling domain of CD28, and the signaling domain of CD137.

As aforementioned either the extracellular part or the transmembrane domain or the cytoplasmic domain of a CAR may also comprise a heterodimerizing domain for the aim of separating key signaling and antigen recognition modules of the CAR onto two (or more) separate polypeptide chains. For this purpose, the antigen binding domains AB1 and AB2 disclosed in the present invention may be incorporated into the two separate polypeptide chains that, when dimerized via the small molecule as disclosed herein, lead to a functional CAR.

The CAR may be further modified to include on the level of the nucleic acid encoding the CAR one or more operative elements to eliminate CAR expressing immune cells by virtue of a suicide switch. The suicide switch can include, for example, an apoptosis inducing signaling cascade or a drug that induces cell death. In one embodiment, the nucleic acid expressing and encoding the CAR can be further modified to express an enzyme such thymidine kinase (TK) or cytosine deaminase (CD). The CAR may also be part of a gene expression system that allows controlled expression of the CAR in the immune cell. Such a gene expression system may be an inducible gene expression system and wherein when an induction agent (e.g. caffeine as disclosed herein) is administered to a cell being transduced with said inducible gene expression system, the gene expression system is induced and said CAR is expressed on the surface of said transduced cell.

In some embodiments, the endodomain may contain a primary cytoplasmic signaling domain or a co-stimulatory region, but not both.

In some embodiments, the CAR may be a "SUPRA" (split, universal, and programmable) CAR, where a "zipCAR" domain may link an intracellular co-stimulatory domain and an extracellular leucine zipper (WO2017/091546). This zipper may be targeted with a complementary zipper fused e.g. to an scFv region to render the SUPRA CAR T cell tumor specific. This approach would be particularly useful for generating universal CAR T cells for various tumors; adapter molecules could be designed for tumor specificity and would provide options for altering specificity post-adoptive transfer, key for situations of selection pressure and antigen escape.

In some embodiments of the present invention the heterodimerizing system as disclosed herein may be applied in the context of a CAR, i.e. the CAR may be split into two separate polypeptide chains, which contain the dimerization domains AB 1 and AB2, respectively, that are disclosed in the present invention. This architecture enables conditional heterodimerization of the two polypeptide chains with the small molecules disclosed herein, i.e. a derivative of xanthine such as caffeine or theophylline, thereby assembling a functional CAR. Specific embodiments of the invention are disclosed herein that apply the heterodimerization system as disclosed herein in CAR settings.

The CARs as described herein may be designed to comprise any portion or part of the above-mentioned domains as described herein in any order and/or combination resulting in a functional CAR, i.e. a CAR that mediated an immune effector response of the immune effector cell that expresses the CAR as disclosed herein.

The term "tagged polypeptide" as used herein refers to a polypeptide that has bound thereto directly or indirectly at least one additional component, i.e. the tag. The tagged polypeptide as used herein is able to bind an antigen expressed on a target cell. The polypeptide may be an antibody or antigen binding fragment thereof that binds to an antigen expressed on the surface of a target cell such as a tumor associated antigen on a cancer cell. The polypeptide of the tagged polypeptide alternatively may be a cytokine or a growth factor or another soluble polypeptide that is capable of binding to an antigen of a target cell.

The terms "adapter" or "adapter molecule" or "tagged polypeptide" as used herein may be used interchangeably.

The tag may be e.g. a hapten or dextran and the hapten or dextran may be bound by the antigen binding domain of the polypeptide, e.g. a CAR, comprising an antigen binding domain specific for the tag.

Haptens such as e.g. FITC, biotin, PE, streptavidin or dextran are small molecules that elicit an immune response only when attached to a large carrier such as a protein; the carrier may be one that also does not elicit an immune response by itself. Once the body has generated antibodies to a hapten-carrier adduct, the small-molecule hapten may also be able to bind to the antibody, but it will usually not initiate an immune response; usually only the hapten-carrier adduct can do this.

However, the tag may also be a peptide sequence e.g. chemically or recombinantly coupled to the polypeptide part of the tagged polypeptide. The peptide may be selected from the group consisting of c-Myc-tag, Strep-Tag, Flag-Tag, and Polyhistidine-tag. The tag may also be streptavidin. The tag portion of the tagged polypeptide is only constrained by being a molecular component that can be recognized and specifically bound by the antigen binding domain specific for the tag of the CAR. For example, when the tag is FITC (Fluorescein isothiocyanate), the tag-binding domain may constitute an anti-FITC scFv. Alternatively, when the tag is biotin or PE (phycoerythrin), the tag-binding domain may constitute an anti-biotin scFv or an anti-PE scFv.

The term "antibody" as used herein is used in the broadest sense to cover the various forms of antibody structures including but not being limited to monoclonal and polyclonal antibodies (including full length antibodies), multispecific antibodies (e.g. bispecific antibodies), antibody fragments, i.e. antigen binding fragments of an antibody, immunoadhesins and antibody-immunoadhesin chimeras, that specifically recognize (i.e. bind) an antigen. "Antigen binding fragments" comprise a portion of a full-length antibody, preferably the variable domain thereof, or at least the antigen binding site thereof ("an antigen binding fragment of an antibody"). Examples of antigen binding fragments include Fab (fragment antigen binding), scFv (single chain fragment variable), single domain antibodies (nanobodies or VHHs, or single VH domains), diabodies, dsFv, Fab', diabodies, single-chain antibody molecules, and multispecific antibodies formed from antibody fragments. The antibody or antibody fragment may be human, fully human, humanized, human engineered, non-human, and/or chimeric. The non-human antibody or antibody fragment may be humanized to reduce immunogenicity to humans, while retaining the specificity and affinity of the parental non-human antibody. Chimeric antibodies may refer to antibodies created through the joining of two or more antibody genes which originally encoded for separate antibodies.

A single-domain antibody (sdAb), also known as a nanobody, is an antibody fragment consisting of a single monomeric variable antibody domain. Like a whole antibody, it is able to bind selectively to a specific antigen. With a molecular weight of only 12-15 kDa, single-domain antibodies are much smaller than common antibodies (150-160 kDa) which are composed of two heavy protein chains and two light chains, and even smaller than Fab fragments (~50 kDa, one light chain and half a heavy chain) and single-chain variable fragments (~25 kDa, two variable domains, one from a light and one from a heavy chain).

The first single-domain antibodies were engineered from heavy-chain antibodies found in camelids; these are

called VHH ($V_HH$) fragments. Cartilaginous fishes also have heavy-chain antibodies (IgNAR, 'immunoglobulin new antigen receptor'), from which single-domain antibodies called V-NAR fragments can be obtained. Alternatively, single-domain antibodies can also be based on human VH or VL domains or mutated versions thereof.

The terms "AB0", "AB1" and "AB2" as used herein refer to antigen binding domains and refer to any portion of an antibody (e.g., whole antibody or fragment thereof) that retains the ability to bind to an intended antigen or epitope. As used herein, AB 1 and AB2 may be derived by (genetic) modification of AB0.

The terms "having specificity for", "specifically binds" or "specific for" with respect to an antigen-binding domain of an antibody, of a fragment thereof or of a CAR refer to an antigen-binding domain which recognizes and binds to a specific antigen, but does not substantially recognize or bind other molecules in a sample. An antigen-binding domain that binds specifically to an antigen from one species may bind also to that antigen from another species. This cross-species reactivity is not contrary to the definition of that antigen-binding domain is specific. An antigen-binding domain that specifically binds to an antigen may bind also to different allelic forms of the antigen (allelic variants, splice variants, isoforms etc.). This cross reactivity is not contrary to the definition of that antigen-binding domain is specific.

As used herein, the term "antigen" is intended to include substances that bind to or evoke the production of one or more antibodies and may comprise, but is not limited to, proteins, peptides, polypeptides, oligopeptides, lipids, carbohydrates such as dextran, haptens and combinations thereof, for example a glycosylated protein or a glycolipid, or a derivative of xanthine such as caffeine or theophylline. The term "antigen" as used herein refers to a molecular entity that may be expressed e.g. on the surface of a target cell and that can be recognized by means of the adaptive immune system including but not restricted to antibodies or TCRs, or engineered molecules including but not restricted to endogenous or transgenic TCRs, CARs, scFvs or multimers thereof, Fab-fragments or multimers thereof, antibodies or multimers thereof, single chain antibodies or multimers thereof, single-domain antibodies or multimers thereof, or any other molecule that can execute binding to a structure with high affinity.

The term "soluble antigen" as used herein refers to an antigen that is not immobilized on surfaces such as beads or cell membranes.

The terms "immune cell" or "immune effector cell" may be used interchangeably and refer to a cell that may be part of the immune system and executes a particular effector function such as T cells, alpha-beta T cells, NK cells, NKT cells, B cells, innate lymphoid cells (ILC), cytokine induced killer (CIK) cells, lymphokine activated killer (LAK) cells, gamma-delta T cells, regulatory T cells (Treg), monocytes or macrophages. Preferentially these immune cells are human immune cells. Preferred immune cells are cells with cytotoxic effector function such as alpha-beta T cells, NK cells, NKT cells, ILC, CIK cells, LAK cells or gamma-delta T cells. Most preferred immune effector cells are T cells and NK cells. Tumor infiltrating lymphocytes (TILs) are T cells that have moved from the blood of a subject into a tumor. These TILs may be removed from a patient's tumor by methods well known in the art, e.g. enzymatic and mechanic tumor disruption followed by density centrifugation and/or cell marker specific enrichment. TILs may be genetically engineered as disclosed herein, and then given back to the patient. "Effector function" means a specialized function of a cell, e.g. in a T cell an effector function may be cytolytic activity or helper activity including the secretion of cytokines.

T cells or T lymphocytes are a type of lymphocyte that play a central role in cell-mediated immunity. They can be distinguished from other lymphocytes, such as B cells and natural killer cells (NK cells), by the presence of a T cell receptor (TCR) on the cell surface. There are several subsets of T cells, each with a distinct function.

T helper cells (TH cells) assist other white blood cells in immunologic processes, including maturation of B cells into plasma cells and memory B cells, and activation of cytotoxic T cells and macrophages. These cells are also known as CD4+ T cells because they express the CD4 glycoprotein on their surface. Helper T cells become activated when they are presented with peptide antigens by MHC class II molecules, which are expressed on the surface of antigen-presenting cells (APCs). Once activated, they divide rapidly and secrete small proteins called cytokines that regulate or assist in the active immune response. These cells can differentiate into one of several subtypes, including TH1, TH2, TH3, TH17, Th9, or TFH, which secrete different cytokines to facilitate a different type of immune response. Signaling from the APC directs T cells into particular subtypes.

Cytotoxic T cells (TC cells, or CTLs) destroy virally infected cells and tumor cells and are also implicated in transplant rejection. These cells are also known as CD8+ T cells since they express the CD8 glycoprotein at their surface. These cells recognize their targets by binding to antigen associated with MHC class I molecules, which are present on the surface of all nucleated cells.

Memory T cells are a subset of antigen-specific T cells that persist long-term after an infection has resolved. They quickly expand to large numbers of effector T cells upon re-exposure to their cognate antigen, thus providing the immune system with "memory" against past infections. Memory T cells comprise three subtypes: central memory T cells (TCM cells) and two types of effector memory T cells (TEM cells and TEMRA cells). Memory cells may be either CD4+ or CD8+. Memory T cells typically express the cell surface protein CD45RO.

Regulatory T cells (Treg cells), formerly known as suppressor T cells, are crucial for the maintenance of immunological tolerance. Their major role is to shut down T cell-mediated immunity toward the end of an immune reaction and to suppress auto-reactive T cells that escaped the process of negative selection in the thymus.

Two major classes of CD4+ Treg cells have been described - Foxp3+ Treg cells and Foxp3-Treg cells.

Natural killer T cells (NKT cells - not to be confused with natural killer cells of the innate immune system) bridge the adaptive immune system with the innate immune system. Unlike conventional T cells that recognize peptide antigens presented by major histocompatibility complex (MHC) molecules, NKT cells recognize glycolipid antigen presented by a molecule called CD1d. Once activated, these cells can perform functions ascribed to both Th and Tc cells (i.e., cytokine production and release of cytolytic/cell killing molecules).

The term "natural killer cells (NK cells)" are defined as large granular lymphocytes (LGL) and constitute the third kind of cells differentiated from the common lymphoid progenitor-generating B and T lymphocytes. NK cells are known to differentiate and mature in the bone marrow, lymph nodes, spleen, tonsils, and thymus, where they then enter the circulation. NK cells differ from natural killer T cells (NKTs) phenotypically, by origin and by respective effector functions; often, NKT cell activity promotes NK cell activity by secreting IFN$\gamma$. In contrast to NKT cells, NK cells do not express T-cell receptors (TCR) or pan T marker CD3 or surface immunoglobulins (Ig) B cell receptors, but they usually express the surface markers CD16 (FcyRIII) and CD56 in humans, NK1.1 orNK1.2 in C57BL/6 mice. Up to 80% of human NK cells also express CD8. Continuously growing NK cell lines can be established from cancer patients and common NK cell lines are for instance NK-92, NKL and YTS.

Immunotherapy is a medical term defined as the "treatment of disease by inducing, enhancing, or suppressing an immune response". Immunotherapies designed to elicit or amplify an immune response are classified as activation immunotherapies, while immunotherapies that reduce or suppress are classified as suppression immunotherapies. Cancer immunotherapy as an activating immunotherapy attempts to stimulate the immune system to reject and destroy tumors. Adoptive cell transfer uses cell-based, preferentially T cell-based or NK cell-based cytotoxic responses to attack cancer cells. T cells that have a natural or genetically engineered reactivity to a patient's cancer are generated *in-vitro* and then transferred back into the cancer patient. If the immune cells have been genetically modified to express a CAR, the immunotherapy is referred to as "CAR cell immunotherapy" or in case of use of T cells only as "CAR T cell therapy" or "CAR T cell immunotherapy".

The term "treatment" as used herein means to reduce the frequency or severity of at least one sign or symptom of a disease.

The terms "therapeutically effective amount" or "therapeutically effective population" mean an amount of a cell population which provides a therapeutic benefit in a subject.

As used herein, the term "subject" refers to an animal. Preferentially, the subject is a mammal such as mouse, rat, cow, pig, goat, chicken dog, monkey or human. More preferentially, the subject is a human. The subject may be a subject suffering from a disease such as cancer (a patient) or from an autoimmune disease or from an allergic disease or from an infectious disease or from graft rejection.

The term "expression" as used herein is defined as the transcription and/or translation of a particular nucleotide sequence driven by its promoter in a cell.

The terms "engineered cell" and "genetically modified cell" as used herein can be used interchangeably. The terms mean containing and/or expressing a foreign gene or nucleic acid sequence which in turn modifies the genotype and/or phenotype of the cell or its progeny. Especially, the terms refer to the fact that cells, preferentially T cells can be manipulated by recombinant methods well known in the art to express stably or transiently peptides or proteins which are not expressed in these cells in the natural state. For example, T cells, preferentially human T cells are engineered to express an artificial construct such as a chimeric antigen receptor (CAR) on their cell surface.

The term "cancer" is known medically as a malignant neoplasm. Cancer is a broad group of diseases involving unregulated cell growth and includes all kinds of leukemia, among many others. In cancer, cells (cancerous cells) divide and grow uncontrollably, forming malignant tumors, and invading nearby parts of the body. The cancer may also spread to more distant parts of the body through the lymphatic system or bloodstream. There are over 200 different known cancers that affect humans.

The terms "nucleic acid", "nucleic acid sequence/molecule'" or "polynucleotide" as used interchangeably herein refer to polymers of nucleotides. Polynucleotides, which can be hydrolyzed into monomeric "nucleotides." The monomeric nucleotides can be hydrolyzed into nucleosides. As used herein, the term "polynucleotides" encompasses, but is not limited to, all nucleic acid sequences which are obtained by any means available in the art, including, without limitation, recombinant means, i.e., the cloning of nucleic acid sequences from a recombinant library or a cell genome, using ordinary cloning technology and PCR, and the like, and by synthetic means.

The term "operably linked" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked DNA sequences are contiguous and, where necessary to join two protein coding regions, in the same reading frame.

Examples

*Example 1: Establishment of the mutant acVHH-V104D as an example for the generation of a first antigen binding domain AB1 according to the present invention*

Expression of soluble nanobodies and preparative size exclusion chromatography.

The sequence of the His tag - acVHH-WT anti-caffeine nanobody (acVHH-WT) was ordered as a gene fragment (Twist) and cloned into a pET21+ expression vector. This was then used as base to obtain the single point mutation variants via site directed mutagenesis (QuikChange Lightning Site-Directed Mutagenesis Kit, Agilent).

4 variants of acVHH-WT were cloned this way and named after their point mutation: V104D (acVHH-V104D), F47D, T50E, F47K.

After verifying the sequences with Sanger sequencing the nanobodies were expressed in *E.coli* Rosetta cells. Cells were cultivated in LB medium (10 g/L peptone, 5 g/L yeast extract, 10 g/L NaCl) supplemented with 100 μg/mL ampicillin overnight at 37°C, 180 rpm. The following day the cells were diluted in the same culture medium to an $OD_{600}$ of 0.2 and further cultivated to an $OD_{600}$ of 0.8-1. Protein expression was induced by addition of 1 mM IPTG and incubation at 20°C and 180 rpm overnight. To harvest the proteins, the cultures were centrifuged at 5000 g for 20 min at 4°C: The cell pellet was resuspended in 30 mL sonication buffer (50 mM phosphate buffer pH 7.5 containing 300 mM NaCl, 3 % glycerol, 1 % Triton X-100) and the suspension was sonicated twice for 2 min (pulse: 1.0 - 1.0 seconds, 90 % amplitude) on ice. The cell debris was removed by an additional centrifugation step at 20 000 g, 30 min at 4°C. The resultant supernatant containing the soluble proteins was supplemented with 10 mM imidazole to prevent unspecific binding during the purification step, and filtered through a 0.45 μm Durapore filter.

Protein purification was carried out using metal affinity chromatography. The filtered supernatant was applied to a HisTrap FF column (GE Healthcare) connected to a Bio-Rad purifier system (Bio-Rad NG-C chromatography system), allowing for binding of the His - tagged acVHH-variants to the Nickel containing column. Unspecific interactions were removed by washing the column (50 mM phosphate buffer pH 7.5 with 500 mM NaCl) and the elution of the acVHH was carried out by applying a linear imidazole gradient (from 5 % to 100 % of a buffer containing 50 mM phosphate, pH 7.5, 500 mM NaCl and 500 mM imidazole). Absorbance at 280 nM was detected and the corresponding fractions were analysed by SDS-PAGE. Fractions containing a protein of the appropriate size (~15 kDa) were pooled and the buffer was exchanged to phosphate buffered saline (PBS, pH 7.4) using a 10 kDa SnakeSkin dialysis tubing (Thermo Scientific) and overnight dialysis at 4°C. Purified proteins in PBS were then concentrated via subsequent centrifugation steps using Amicon Ultra-15 10 K centrifugal filters (Merck Millipore).

Protein aggregates were removed via preparative size exclusion chromatography (SEC). The samples were first centrifuged at 16 000 rpm for 3 min to remove large aggregates and the supernatant was loaded onto a HiLoad Superdex 75 pg column connected to a Bio-Rad NG-C chromatography system. Elution was performed in PBS and the fractions containing proteins based on absorbance at 280 nM were pooled, concentrated as mentioned above and stored at - 30°C.

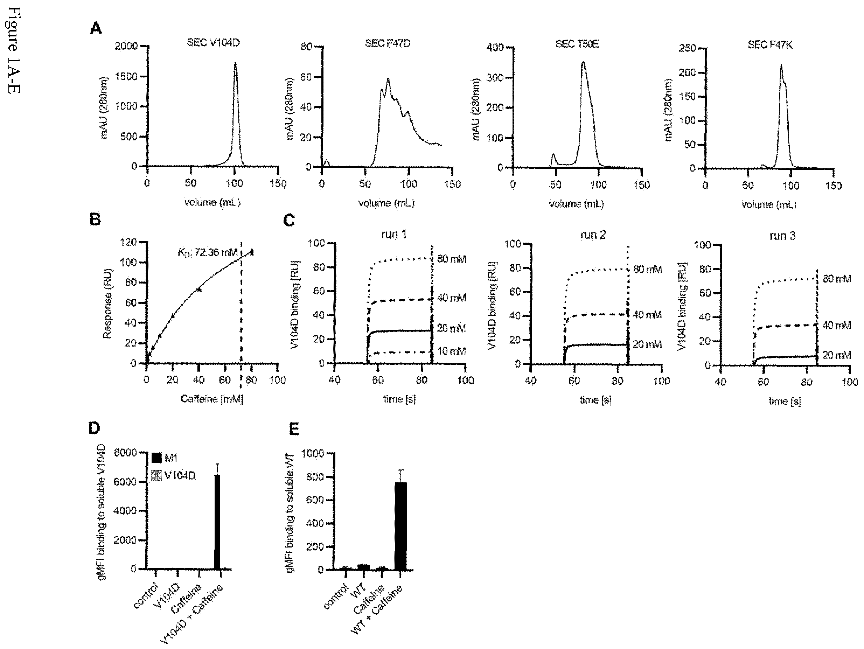

Figure 1A shows the preparative SEC profiles of the 4 nanobodies (acVHH variants) containing the respective single point mutations. These results show that, among those 4 acVHH variants, only acVHH-V104D elutes as a defined monomeric peak. All the three other mutants show partial elution at earlier retention volumes, indicating at least partial aggregation and/or oligomerization. Thus, based on these results, we chose acVHH-V104D as the AB 1 variant for the next experiments. These results also demonstrate that the generation of a first antigen binding domain AB 1 according to the present invention through single point mutations in the dimerization interface of the caffeine-induced dimeric structure (PDB-ID 6QTL) is not trivial, yielding several partially aggregated or oligomerized variants. However, acVHH-V104D showed perfect monomeric behaviour and was therefore selected for further experiments.

Characterisation of the interaction between acVHH-V104D and caffeine by SPR

Surface plasmon resonance (SPR) was used to determine the binding affinity ($K_D$) of caffeine to acVHH-V104D using a BiacoreT200 (GE Healthcare). A stock of 30 μg/mL protein acVHH-V104D was prepared in 10 mM NaAc buffer of pH 5 and covalently immobilised on a CM5 chip (GE Healthcare) with a 300 sec contact time and 10 μL/min flow rate to reach 4 600 response units (RU). Multi-cycle kinetics experiments (MCK) were performed where caffeine in PBS was titrated on the surface of the chip with a flow rate of 30 μL/min, contact time of 30 sec and dissociation time of 120 sec. $K_D$ values were obtained by steady state analysis.

Figure 1B shows the steady state analysis of the MCK experiments of Figure 1C, demonstrating that caffeine binds with only very low affinity to acVHH-V104D ($K_D$ = 72.36 mM).

Yeast surface display analysis of acVHH-V104D homodimerization.

The acVHH variant coding inserts were amplified by PCR (917_u: see SEQ ID NO:9 and 918_d: see SEQ ID NO:10, those primers were used for all clonings into the pCTCON2V vector) and cloned into the linearized yeast surface display pCTCON2V (which is an in house version of the well-described yeast display vector pCTCON2; Traxlmayr et al., J Biol Chem. 2016 Oct 21;291(43):22496-22508) vector via Gibson assembly (NEBuilder HiFi DNA Assembly Master Mix, NEB).

pCTCON2V vector linearization was carried out via overnight digestions with SalI, NheI and BamHI (NEB) at 37°C. The linearized vector was then purified via ethanol purification and dissolved in $H_2O$ to a proper concentration.

After Gibson assembly the acVHH variant encoding plasmids were heat shock transformed into XL-10 *E.coli* cells and the isolated plasmids were sent to Microsynth for sequencing. The different domains of the resulting fusion proteins are as follow: Aga2p - HA-tag - $(Gly_4Ser)_3$ linker - acVHH - c-myc tag as in SEQ ID NO:11 where acVHH is acVHH-WT. All other sequences for yeast surface expression are analogous, with acVHH-WT being exchanged to the other nanobody variants, respectively.

Plasmids encoding for correct sequences of acVHH variants were then transformed into the *S. cerevisiae* strain EBY100 with the Frozen-EZ Yeast Transformation II Kit (Zymo Research) and plated on selective SD-CAA plates (20 g/L glucose, 6.7 g/L yeast nitrogen base, 5 g/L bacto casamino acids, 11.85 g/L sodium citrate dihydrate, 7.4 g/L citric acid monohydrate, 15 g/L agar, 182 g/L sorbitol) to allow for growth of transformants. Individual colonies were used to inoculate SD-CAA medium (20 g/L glucose, 6.7 g/L yeast nitrogen base, 5 g/L bacto casamino acids, 11.85 g/L sodium citrate dihydrate and 7.4 g/L citric acid monohydrate) overnight at 30°C, 180 rpm. The next day the cultures were diluted to an $OD_{600}$ of 0.2 and monitored via absorbance at 280 nm. Upon reaching an $OD_{600}$ of 0.8-1 the cells were pelleted via 2 000 g 5 min centrifugation and acVHH variant expression was induced by resuspending the cells in SG-CAA (20 g/L galactose, 2 g/L glucose, 6.7 g/L yeast nitrogen base, 5 g/L bacto casamino acids, 10.2 g/L $Na_2HPO_4$*7 $H_2O$, 8.56 g/L $NaH_2PO_4$*$H_2O$) and incubating them overnight at 20°C, 180 rpm.

The staining for flow cytometry characterization was performed in PBSA (PBS containing 15 μM bovine serum albumin). Cells displaying either acVHH-V104D, acVHH-WT or acVHH-M1 were incubated with caffeine and/or the soluble interaction partner (acVHH-V104D or acVHH-WT) for 1 h at 4°C with shaking. In addition, controls with buffer only (containing neither an acVHH variant nor caffeine) were included. After a washing step the cells were stained for 20 min at 4°C and shaking with either streptavidin-Alexa Fluor 647 (Invitrogen) or Penta-His-Alexa Fluor 647 (Qiagen) to assess acVHH-V104D or acVHH-WT binding, as well as anti-HA-Alexa Fluor 488 (clone 16B12, Biolegend) or anti-c-myc AlexaFluor 488 (9E10, Thermofisher) to assess display (anti-HA) or full-length display (anti-c-myc) of the acVHH variants, respectively. After a final washing step, the cells were analyzed with a CytoFLEX S flow cytometer (Beckman Coulter).

Figure 1D shows binding of yeast cells displaying either acVHH-V104D (grey bars) or acVHH-M1 (black bars) to 500 nM soluble acVHH-V104D in the presence or absence of 10 μM caffeine, demonstrating that acVHH-V104D does not homodimerize to detectable levels even in the presence of caffeine. M1 is a positive control generated in Example 2. Thus, these data demonstrate that acVHH-V104D heterodimerizes with acVHH-M1 in the presence of caffeine, but it does not homodimerize (with itself) to detectable levels in the presence of caffeine.

Figure 1E shows binding of acVHH-WT displayed on yeast to 500 nM soluble acVHH-WT in the presence or absence of 10 μM caffeine, confirming that acVHH-WT homodimerizes in the presence of caffeine.

Thus, together, Figure 1D and 1E demonstrate that the homodimerization of acVHH-WT observed in the presence of caffeine was eliminated by introduction of the mutation V104D. These findings are further supported in Example 3 described below.

*Example2: Engineering of dimerization partners of acVHH-V104D, as examples for the generation of a second antigen binding domain AB2 according to the present invention*

Establishment of the yeast surface display library

An acVHH gene fragment with 8 NNK randomised positions comprising SEQ ID NO:3 was ordered (Twist) and additionally submitted to error prone PCR mutagenesis using the GeneMorph II- random mutagenesis kit (Agilent) with the following parameters: 100 ng target DNA, 20 cycles. Following amplification and subsequent gel purification (Qiaquick Gel Extraction Kit, Qiagen) the inserts were re-amplified via standard PCR to obtain a high concentration and subjected to ethanol (EtOH) purification.

Electrocompetent *S. cerevisiae* cells (strain EBY100) were prepared according to the protocol published by Chen et al. (Chen et al., Methods Enzymol. 2013;523:303-26). Electrocompetent yeast cells were prepared fresh on the day of the electroporation. Cells were plated a few days prior to electroporation on a YP-agar plate (20 g/L peptone, 10 g/L yeast extract, 15 g/L agar, 20 g/L glucose) and 1 colony was cultivated in YPD-medium overnight (YP-agar without agar), shaking with 180 rpm at 30°C. The grown culture was diluted the next morning to an $OD_{600}$ of 0.2 in 50 mL per 2 electroporations, and further cultivated in YPD at 30°C until it reached an $OD_{600}$ of 1.3-1.5. Cells were pelleted at 3 000 rpm for 3 min and the supernatant was resuspended in 25 mL of a freshly prepared 100 mM lithium acetate, 10 mM DTT solution. Following a 10 min incubation at 30°C, the cells were again pelleted and washed with 25 mL chilled $H_2O$. After another round of centrifugation, the pellet was resuspended in 300 μL chilled $H_2O$ so that the total volume equals 500 μL and the 10 μL ethanol purified insert and 2 μg of the linearized pCTCON2V vector were added. 250 μL of the cell-DNA preparation were added to pre-chilled 2 mm electroporation cuvettes and the electroporation was carried out on a Bio-Rad Gene Pulser Xcell electoporator with the square wave protocol, 500 V and a single pulse of 15 ms (Chao et al., Nat Protoc. 2006;1(2):755-768;Chen et al., Methods Enzymol. 2013;523:303-26). The cells were immediately rescued with 1 mL pre-warmed YPD and incubated at 30°C for 1 h. A library diversity of $1.3\times10^8$ transformants was calculated by plating serial dilutions of the cells immediately after the 1 h-incubation on selective SD-CAA plates and allowing for growth of transformants. The remaining cells were pelleted at 3 000rpm and 3 min, resuspended in selective SD-CAA medium and incubated at 30°C, 180 rpm.

Screening for acVHH-V104D binding interaction partners (i.e., AB2 according to the present invention) using yeast surface display (YSD)

The day after electroporation of yeast, the library was sub-cultivated to an $OD_{600}$ of 1 in SD-CAA and incubated at 30°C, 180 rpm. Upon reaching an $OD_{600}$ of 4, expression of the acVHH library on the surface of the yeast cells was induced by centrifuging the cells and resuspending them in SG-CAA medium (20 g/L galactose, 2 g/L glucose, 6.7 g/L yeast nitrogen base, 5 g/L bacto casamino acids, 10.2 g/L $Na_2HPO_4$*7 $H_2O$, 8.56 g/L $NaH_2PO_4$*$H_2O$). Cells were then incubated at 20°C overnight with shaking. To improve specificity of the library and remove variants presenting no or very low affinity binding to acVHH-V104D, two cycles of magnetic bead selection were performed with biotinylated acVHH-V104D (EZ-Link Sulfo-NHS-LC-LC-Biotin kit, Thermo Fisher Scientific) loaded on magnetic streptavidin-coated Dynabeads (Life Technologies) (Angelini et al., Methods Mol Biol. 2015;1319:3-36; Chen et al., Methods Enzymol. 2013;523:303-26). The first cycle consisted of a positive selection where cells displaying variants that could bind to bead-loaded acVHH-V104D in the presence of 10 μM caffeine were kept and non-functional or low affinity variants were washed off. The second cycle included two negative selections as well as another positive selection. In negative selection rounds, cells displaying variants that could bind to bare beads (i.e., not loaded with biotinylated acVHH-V104D) were discarded to prevent enrichment of streptavidin-specific or unspecific binders.

After these bead selection rounds the library was further enriched by fluorescence activated cell sorting (FACS). The yeast display library was incubated with 500 nM soluble acVHH-V104D for 1 h at 4°C while shaking in the presence or absence of 10 μM caffeine in PBSA (PBS supplemented with 15 μM bovine serum albumin). Biotinylated and non-biotinylated $His_6$-tagged acVHH-V104D were used in different selection rounds to prevent selection of biotin-specific binders. After a washing step with PBSA, the cells were stained for 20 min at 4°C and shaking with either streptavidin-Alexa Fluor 647 (Invitrogen) or Penta-His-Alexa Fluor 647 (Qiagen) to assess acVHH-V104D binding, as well as anti-HA-Alexa Fluor 488 (clone 16B12, Biolegend) or anti-c-myc AlexaFluor 488 (9E10, Thermofisher) to assess display (anti-HA) or full-length display (anti-c-myc) of the acVHH variants, respectively. After a final washing step with PBSA, cells were sorted using a SONY Sorter SH800.

Positive and negative FACS selection rounds were performed in an alternating manner. In positive selections, cells displaying acVHH variants that could bind to soluble acVHH-V104D in the presence of caffeine were sorted, whereas in negative selections the sorted cells where those displaying variants that could not bind to acVHH-V104D in the absence of caffeine. In one case, the library was split based on binding intensity with "high" referring to cells displaying acVHH variants that could bind with a higher intensity than the "low" ones. In total 8 subsequent rounds of FACS were performed.

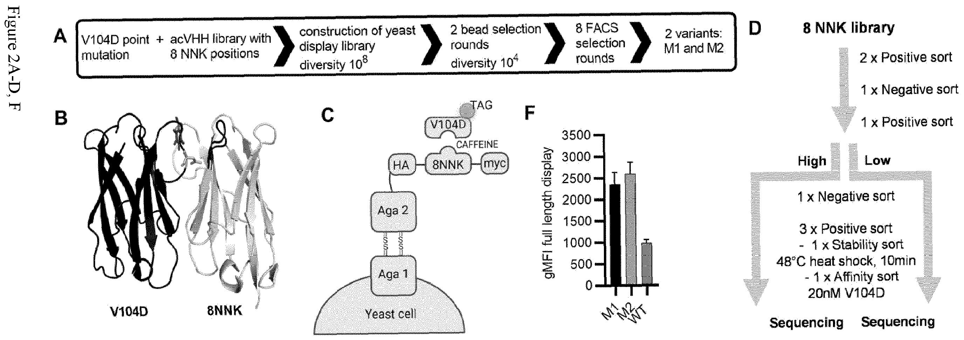

Figure 2A shows a schematic of the engineering process.

Figure 2B represents a model of acVHH-V104D binding to acVHH-8NNK, where acVHH-V104D is the nanobody on the left in black, the V104D point mutation is shown in light grey sticks and caffeine is shown in dark grey sticks. To generate this model with PyMOL, the homodimeric structure of acVHH-WT (PDB-ID 6QTL) was used. The 8NNK library (acVHH-8NNK) is shown in grey on the right and the mutated 8 amino acid loop is colored in black, illustrating that the mutated loop in the 8NNK library (acVHH-8NNK) is in close proximity to the V104D mutation on the other nanobody (acVHH-V104D).

Figure 2C shows a schematic of the yeast surface display platform used for the engineering process. The acVHH-8NNK library is displayed on the surface of the yeast between a HA- and c-myc tag for analysis of expression and full-length expression, respectively. Tag-V104D (tag-acVHH-V104D) is added solubly.

Figure 2D shows the different rounds of FACS sorting.

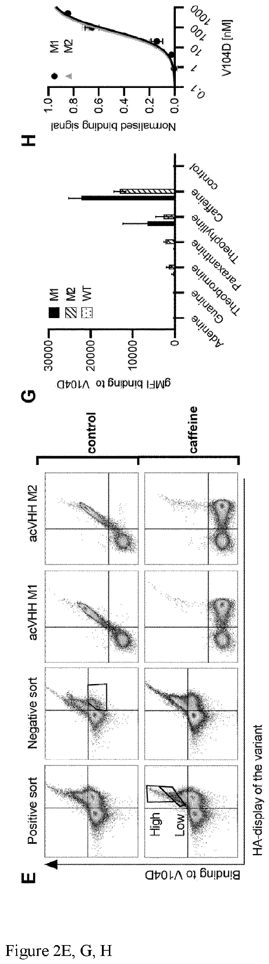

Figure 2E represents representative yeast display flow cytometry plots. In these plots, expression on the yeast surface is shown on the x-axis and binding to acVHH-V104D is shown on the y-axis. In the left column, flow cytometry plots of a representative positive sort are shown, where the cells that could bind to acVHH-V104D in the presence of caffeine were sorted in "high" or "low" gates. In the second column from the left, a representative negative sort is shown, where cells displaying acVHH variants that could not bind to acVHH-V104D in the absence of caffeine (labeled as "control") were sorted.

Yeast surface display characterization of acVHH-V104D interaction partners.

48 clones were sequenced after the last selection round. Plasmids were obtained from the yeast library via a Zymoprep yeast plasmid miniprep kit II (Zymo Research) and the resulting DNA pool was electroporated into *E.coli* 10-beta electrocompetent cells (NEB) according to the supplier's protocol and plated on LB ampicillin plates. On the next day, 48 single colonies were used to inoculate 48 wells with LB supplemented with ampicillin in a Microsynth sequencing plate and sequenced using the primer: 5-CGTTTGTCAGTAATTGCGGTTCTC (SEQ ID NO: 12). 2 variants were obtained, termed acVHH-M1 and acVHH-M2. Plasmids encoding for these two acVHH variants of interest were then transformed into the *S. cerevisiae* strain EBY100 with the Frozen-EZ Yeast Transformation II Kit (Zymo Research).

Expression of the acVHH variants on yeast was induced by culturing the cells in SG-CAA medium (20 g/L galactose, 2 g/L glucose, 6.7 g/L yeast nitrogen base, 5 g/L bacto casamino acids, 10.2 g/L $Na_2HPO_4*7\ H_2O$, 8.56 g/L $NaH_2PO_4*H_2O$) overnight at 20°C and 180 rpm. Detection of full-length proteins on the surface of the cells was carried out by staining the cells with anti-c-myc AlexaFluor 488 (9E10, Thermo Fisher). To measure binding to soluble acVHH-V104D, the cells were incubated with soluble acVHH-V104D in the presence of buffer only (PBSA, "control") or in the presence of PBSA supplemented with 10μM caffeine, theobromine, theophylline, paraxanthine, adenine or guanine. After a washing step with PBSA, they were stained with an anti-HA-Alexa Fluor 488 (BioLegend) to gate for displaying yeast cells and the geometric mean fluorescence intensity (gMFI) of binding to acVHH-V104D, detected by co-staining with a Penta-His-Alexa Fluor 647 (Qiagen) (recognizing the His-tag on acVHH-V104D) was measured.

For $K_D$ measurements, yeast displaying the 2 acVHH variants were titrated with soluble acVHH-V104D in the presence of 10 μM caffeine. $K_D$ values were calculated by fitting the gMFI binding values (for acVHH-V104D binding) to a 1:1 binding interaction model (Zajc et al., Methods Mol Biology. 2022; 2491:155-173).

Figure 2E shows flow cytometry plots representing yeast-displayed acVHH-M1 (third column from the left) and yeast-displayed acVHH-M2 (right column) binding to soluble acVHH-V104D. These data show a clear dependency of the heterodimerization between acVHH-M1 and acVHH-V104D and also of the heterodimerization between acVHH-M2 and acVHH-V104D on the presence of caffeine. Therefore, acVHH-M1 and acVHH-M2 represent two examples for a second antigen binding domain (AB2) according to the present invention.

Figure 2F shows full length display of the engineered variants, measured with yeast display (n=3, averages ± standard deviations). These data demonstrate that the engineered variants acVHH-M1 and acVHH-M2 are well-expressed on the yeast surface, even at higher levels than the original variant acVHH-WT.

Figure 2G shows binding of three yeast-displayed acVHH variants (acVHH-WT, acVHH-M1 and acVHH-M2) to 500 nM soluble acVHH-V104D ± 10 μM compound as indicated, measured with yeast display (n=3, averages ± standard deviations). These data demonstrate that various derivatives of xanthine, including caffeine, theophylline, paraxanthine and theobromine can trigger these heterodimerizations between acVHH-V104D and acVHH-M1 or between acVHH-V104D and acVHH-M2. These data also demonstrate that these heterodimerization systems are most efficient with caffeine. Moreover, these data also show that acVHH-WT does not yield any detectable signal when analyzed for binding to acVHH-V104D even in the presence of caffeine. Finally, both heterodimeric switches (acVHH-V104D + acVHH-M1 or acVHH-V104D + acVHH-M2) are highly specific, as demonstrated by the virtual absence of any background signal in the absence of any small molecule ("control" condition).

Figure 2H shows binding of the acVHH-M1 and acVHH-M2 to increasing concentrations of soluble acVHH-V104D in the presence of 10 μM caffeine. Measured with yeast display and normalized to maximum binding signal (n=3, averages ± standard deviations).

*Example3. Engineering disulfide-free variants of the second antigen binding domain AB2 for intracellular applications*

Characterization of disulfide-free acVHH-M1 variants

acVHH-M1 in pCTCON2V was subjected to site directed mutagenesis (QuikChange Lightning kit, Agilent) with the following primers to exchange the cysteines to other amino acids:

| Variant | Fwd primer | Rev primer |
|---|---|---|
| M1 C22W | Ctgagactgagctggaccgcctctggcag (SEQ ID NO:13) | Ctgccagaggcggtccagctcagtctcag (SEQ ID NO:14) |

(continued)

| Variant | Fwd primer | Rev primer |
|---|---|---|
| M1 C22A | Tctctgagactgagcgctaccgcctctggcag (SEQ ID NO: 15) | Ctgccagaggcggtagcgctcagtctcagaga (SEQ ID NO:16) |
| M1 C22V | Gatctctgagactgagcgttaccgcctctggcagaa (SEQ ID NO:17) | Ttctgccagaggcggtaacgctcagtctcagagatc (SEQ ID NO:18) |
| M1 C22G | Ctctgagactgagcggtaccgcctctggc (SEQ ID NO:19) | Gccagaggcggtaccgctcagtctcagag (SEQ ID NO: 20) |
| M1 C22F | Ctctgagactgagctttaccgcctctggcag (SEQ ID NO: 21) | Ctgccagaggcggtaaagctcagtctcagag (SEQ ID NO: 22) |
| M1 C96A | Accgccgtgtactacgccacagccacaagagc (SEQ ID NO:23) | Gctcttgtggctgtggcgtagtacacggcggt (SEQ ID NO: 24) |
| M1 C96V | Caccgccgtgtactacgtcacagccacaagagcc (SEQ ID NO:25) | Ggctcttgtggctgtgacgtagtacacggcggtg (SEQ ID NO:26) |
| M1 C96P | Accgccgtgtactaccccacagccacaagagc (SEQ ID NO:27) | Gctcttgtggctgtggggtagtacacggcggt (SEQ ID NO: 28) |
| M1 C96W | Gccgtgtactactggacagccacaagagc (SEQ ID NO: 29) | Gctcttgtggctgtccagtagtacacggc (SEQ ID NO:30) |

The following combinations (i.e. acVHH variants) were generated: M1 ΔAA, M1 ΔAV, M1 ΔAW, M1 ΔFA, M1 ΔGW, M1 ΔVA, M1 ΔWA, M1 ΔWP and the sequences were confirmed by Sanger sequencing (Microsynth). For example, the variant M1 ΔAV represents a variant of acVHH-M1, in which the two Cys-residues of the conserved disulfide bond were exchanged to Ala at position 22 and to Val at position 96, respectively. All plasmids were then transformed into the *S. cerevisiae* strain EBY100 with the Frozen-EZ Yeast Transformation II Kit (Zymo Research).

Expression of the variants on yeast was carried out in SG-CAA as described above. Full length display was assessed by staining with an anti-c-myc AlexaFluor 488 (9E10, Thermo Fisher) and binding to soluble acVHH-V104D was assessed by staining with Penta-His-Alexa Fluor 647 (Qiagen).

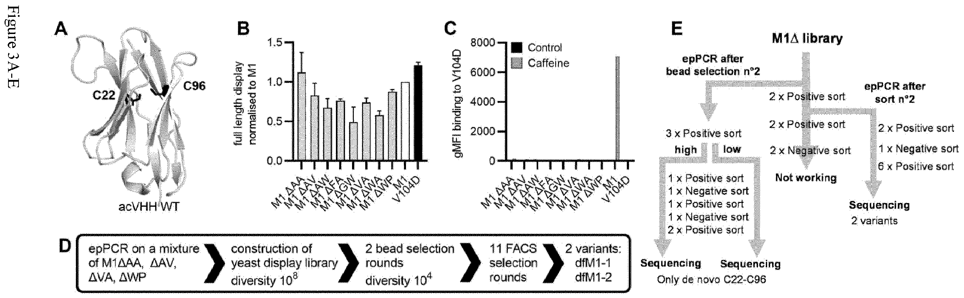

Figure 3A shows a cartoon representation of acVHH-WT (PDB-ID 6QTL) where the cysteines C22 and C96 are highlighted in black. Figure 3B shows full-length display levels on yeast cells of M1 variants with the cysteines exchanged as indicated, normalized to acVHH-M1 (n=3, averages ± standard deviations). Figure 3C shows binding of the acVHH-M1 variants to 500 nM soluble acVHH-V104D ± 10 μM caffeine (n=1). Only the cysteine-containing acVHH-M1 binds to acVHH-V104D in the presence of caffeine. Since none of these disulfide-free acVHH-M1-variants bound to acVHH-V104D even in the presence of caffeine (i.e. none of them was functional in a heterodimerizing switch with acVHH-V104D), four of the best expressed variants (M1 ΔAA, M1 ΔAV, M1 ΔVA, M1 ΔWP) were chosen for further engineering.

Establishment of the yeast surface display library for stability engineering

Inserts coding for M1 ΔAA, M1 ΔAV, M1 ΔVA, M1 ΔWP were amplified by PCR, pooled in an equimolar ratio and subjected to error-prone PCR (GeneMorph II - random mutagenesis kit (Agilent), 100 ng target DNA, 20 cycles). The following steps for insert and vector preparation as well as electroporation were similar to those described above in Example 2 (section "establishment of the yeast surface display library"). A library diversity of $10^8$ transformants was obtained. In this case, also 2 rounds of bead selections, followed by several FACS rounds were performed as in Example 2. After the bead selections, the library was processed in 3 different ways (strategies). The first strategy was to classically sort it for 6 rounds via FACS as described in Example 2. The second strategy was to add another round of error-prone PCR mutagenesis after the 2$^{nd}$ round of FACS and proceed with 9 FACS rounds. For the third strategy, another round of error-prone PCR mutagenesis was added directly after the bead selection rounds and followed with 9 FACS rounds. Positive and negative FACS selections were alternated. Figure 3D represents the full engineering strategy and Figure 3E shows the FACS selections process.

Yeast surface display characterization of disulfide free acVHH-V104D interaction partners

56 clones of the library were sequenced as explained in Example 2. Only 2 variants were obtained, namely dfM1-1 and dfM1-2. Their binding and affinity to soluble acVHH-V104D as well as full length display was assessed with yeast surface display as described in Example 2.

$EC_{50}$ values were calculated like the $K_D$ values by fitting the gMFI binding values to a 1:1 binding interaction model (Zajc et al., Methods Mol Biology. 2022; 2491:155-173).

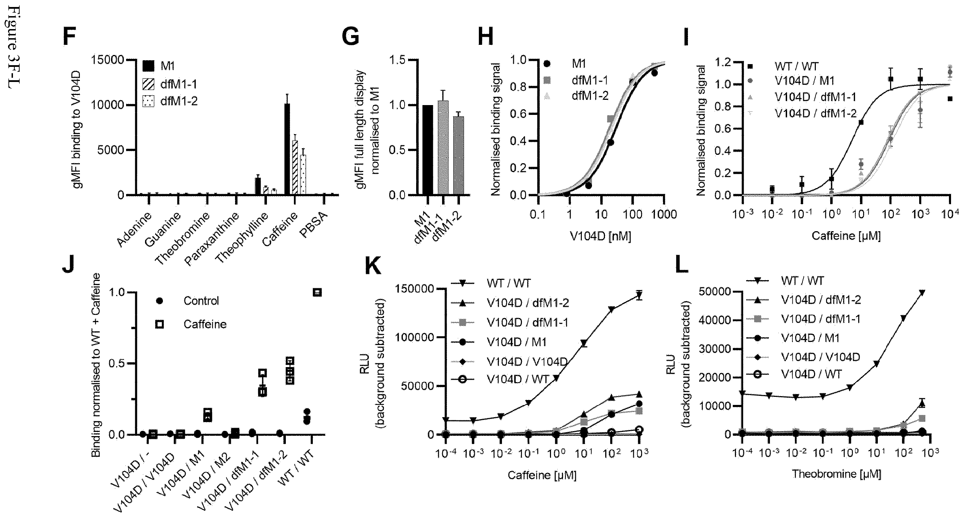

Figure 3F shows binding of acVHH-M1 and its disulfide free variants dfM1-1 and dfM1-2 to 500 nM soluble acVHH-V104D $\pm$ 10 $\mu$M compound as indicated, measured with yeast display (n=3, averages $\pm$ standard deviations). These data demonstrate that these heterodimeric switches can be triggered with caffeine or theophylline and that caffeine is more efficient. Moreover, these data again confirm the high specificity of these switches, since buffer only ("PBSA") does not trigger any detectable signal.

Figure 3G shows the full-length display of acVHH-M1 and its disulfide free variants, measured with yeast display and normalized to acVHH-M1 (n=3, averages $\pm$ standard deviations).

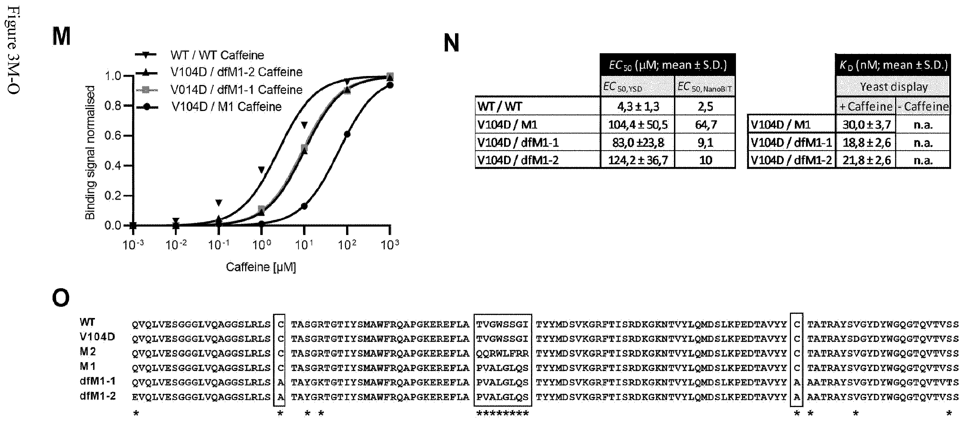

Figure 3H shows binding of acVHH-M1 and the disulfide free M1 variants dfM1-1 and dfM1-2 to increasing concentrations of soluble acVHH-V104D in the presence of 30 $\mu$M caffeine, as measured with yeast display and normalized to maximum binding signal (n=3, averages $\pm$ standard deviations). These data demonstrate that all three acVHH variants (M1, dfM1-1 and dfM1-2) bind to acVHH-V104D with an affinity ($K_D$) in the low nM range. The corresponding $K_D$ values obtained from these binding curves are shown in Fig. 3N, table on the right.

Figure 3I shows binding of yeast-displayed acVHH-M1 or of yeast-displayed disulfide free M1 variants dfM1-1 and dfM1-2 to 400 nM soluble acVHH-V104D, as well as binding of yeast displayed acVHH-WT to 400 nM soluble acVHH-WT. All measurements were performed in the presence of increasing concentrations of caffeine as indicated. Measured with yeast display and normalized to maximum binding signal (n=3, averages $\pm$ standard deviations). These data clearly demonstrate that the $EC_{50}$ values of the heterodimeric switches (V104D+M1 or V104D+dfM1-1 or V104D+dfM1-2) for caffeine are increased when compared with that of the homodimeric switch based on acVHH-WT. In other words, the sensitivity of these novel engineered heterodimeric switches to caffeine was reduced, because higher caffeine concentrations are needed to trigger these heterodimeric switches.

*In vitro* transcription and electroporation of mRNA

The acVHH variants were N-terminally fused to an extracellular globular domain used to assess expression, as well as a transmembrane domain, and C-terminally fused to a subunit of the split NanoLuc (NanoBiT® system), Promega. Thus, the resulting fusion protein consisted of an extracellular globular domain, followed by a transmembrane domain and intracellular acVHH variants followed by a subunit of split NanoLuc luciferase.

The different chains built were as follows: the first polypeptide chain comprised truncated EGFR extracellular and transmembrane domain, followed by a 2xG4S linker and either acVHH-V104D or acVHH-WT, followed by a linker (SEQ ID:31) and 11S NanoBiT® "Large BiT", whereas the second polypeptide chain comprised truncated HER2 extracellular and transmembrane domain, followed by a linker (SEQ ID:32) and either acVHH-V104D or acVHH-WT or acVHH-M1 or acVHH-M2 or dfM1-1 or dfM1-2, followed by a linker (SEQ ID:33) and 114 NanoBiT® "Small BiT". The sequence of the truncated HER2 extracellular and transmembrane domain involved the amino acids at positions 339-683 according to the deposited Uniprot sequence (ID P04626). The sequence of the truncated EGFR extracellular and transmembrane domain involved the amino acids at positions 334-675 according to the deposited Uniprot sequence (ID P00533). The principle of the assay is as follows: upon binding of (i.e. dimerization) of the respective acVHH variants, the fused NanoLuc luciferase subunits are brought into close proximity (the two subunits being fused to the two different polypeptide chains, respectively), thus resulting in a functional luciferase enzyme, whose activity can be measured.

The constructs were constructed by Gibson Assembly (NEB), using 0.02-0.5 pmol of 2-3 PCR amplified DNA fragments. The resulting constructs were amplified by PCR and subsequently used for in vitro transcription.

In vitro transcription was performed with 200 ng of purified PCR product using the mMessage mMachine T7 Ultra Kit (Ambion) according to the manufacturer's instructions. The resulting mRNAs were column purified with the RNeasy Kit (Qiagen), aliquoted to 5 $\mu$g and frozen at - 80°C until electroporation. Jurkat T cells were electroporated with 5 $\mu$g mRNA per chain (10 $\mu$g mRNA total) using the Gene Pulser (Bio-Rad) and the following parameters: square wave protocol, 500 V, 5 ms, 4 mm cuvettes.

Intracellular binding characterized with a protein complementation assay

Intracellular function of the dimers was assessed with the Nano-Glo® Luciferase Assay System (Promega). 16 h after mRNA electroporation, cells expressing the appropriate chains were cultured for 20 min at 37°C in the absence or presence of increasing caffeine or theobromine concentrations. The Nano-Glo reagents were reconstituted as suggested by the manufacturer, added to the cells and luminescence was immediately measured with an ENSPIRE plate reader. $EC_{50}$ values were calculated by fitting the RLU binding values to a 1:1 binding interaction model (Zajc et al., Methods Mol Biology. 2022; 2491:155-173) after subtracting the background signal (Jurkat T cells not electroporated but cultured with the highest concentration of the appropriate compound, i.e. buffer only or 1 mM caffeine or 500 μM theobromine).

Figure 3J shows intracellular binding +/- 30 μM caffeine measured with the NanoBiT® protein complementation assay (Promega) in Jurkat T cells and normalized to the acVHH-WT homodimer in the presence of caffeine (n=3, averages ± standard deviations). These data demonstrate reveal several important findings: (i) acV-HH-V104D does not show any detectable homodimerization even in the presence of caffeine, thus further supporting the yeast display data shown in Figure 1D (i.e., lack of homodimerization of acVHH-V104D). (ii) acVHH-WT shows homodimerization, which is enhanced by addition of caffeine, thus confirming previously published data. (iii) Heterodimerization between acVHH-V104D and acVHH-M1 is weak, also in the presence of caffeine and heterodimerization between acVHH-V104D and acVHH-M2 is not detectable in the presence of caffeine, demonstrating that the disulfide containing variants acVHH-M1 and acVHH-M2 are either not functional or only show weak function when being expressed in the reducing environment of the cytoplasm (because their disulfide bonds cannot be formed properly). In contrast, both dfM1-1 and dfM1-2 (which are disulfide-free mutants of acVHH-M1) show efficient heterodimerization with acVHH-V104D, which is strongly dependent on the presence of caffeine. (iv) The background signal in the absence of caffeine is strongly reduced for all heterodimeric combinations (acVHH-V104D/acVHH-M1; acVHH-V104D/acVHH-M2; acVHH-V104D/dfM1-1; acVHH-V104D/dfM1-2) when compared with acVHH-WT/acVHH-WT homodimers, demonstrating that these heterodimeric switches are less leaky.

Figure 3K shows intracellular binding of the acVHH variant dimers in the presence of increasing concentrations of caffeine measured with the NanoBiT® protein complementation assay (Promega) in Jurkat T cells (n=1, averages ± standard deviations of technical duplicates measured within one experiment). Figure 3M shows the normalized binding signal and the fitted $EC_{50}$ curves for the intracellular protein complementation assay shown in Figure 3K. The data in Figures 3K and 3M reveal several important findings: (i) acVHH-V104D does not show any detectable homodimerization even in the presence of high concentrations of caffeine, thus further supporting the yeast display data shown in Figure 1D and the data shown in Figure 3J (i.e., lack of homodimerization of acVHH-V104D). (ii) acVHH-WT shows homodimerization, which is enhanced by addition of caffeine, thus confirming previously published data and data shown in Figure 3J and 3I. (iii) The background signal at very low concentrations of caffeine is strongly reduced for all heterodimeric combinations (acVHH-V104D/acVHH-M1; acVHH-V104D/dfM1-1; acVHH-V104D/dfM1-2) when compared with acVHH-WT/acVHH-WT homodimers. (iv) acVHH-V104D only shows very weak heterodimerization with acVHH-WT, even at very high concentrations of caffeine, demonstrating that the V104D mutation impairs both homodimerization of acVHH-V104D/acVHH-V104D and also heterodimerization of acVHH-V104D/acVHH-WT in the presence of caffeine. (v) The $EC_{50}$ values of the heterodimeric switches (V104D+M1 or V104D+dfM1-1 or V104D+dfM1-2) for caffeine are increased when compared with that of the homodimeric switch based on acVHH-WT (the $EC_{50}$ values obtained from the curves in Figures 3K and 3M are shown in Figure 3N). In other words, the sensitivity of these novel engineered heterodimeric switches to caffeine was reduced, because higher caffeine concentrations are needed to trigger these heterodimeric switches, thus confirming the data from the yeast display assay shown in Figure 3I and 3N.

Figure 3L shows intracellular binding of the acVHH variant dimers in the presence of increasing concentrations of theobromine measured with the NanoBiT® protein complementation assay (Promega) in Jurkat T cells (n=1, averages ± standard deviations of technical duplicates measured within one experiment). These data show that in addition to caffeine, also other derivatives of xanthine (in this case theobromine) can trigger the engineered heterodimeric switches acVHH-V104D/dfM1-1, as well as acVHH-V104D/dfM1-2.

Figure 3N recapitulates $EC_{50}$ and $K_D$ values for all variants and different types of experiments. $EC_{50}$ values differ between experiments but in all cases the heterodimers have a lower caffeine sensitivity (i.e. higher $EC_{50}$ values) compared to the original homodimer. $EC_{50}$ values are derived from the data shown in Figure 3I (Yeast display) and Figure 3M (NanoBiT), respectively, whereas the $K_D$ values are derived from the data shown in Figure 3H.

Figure 3O shows the acVHH variant sequences. Point mutations compared to the WT sequence are indicated with *. Boxes indicate engineering spots, either the cysteines or the 8 amino acids (8NNK) loop.

*Example 4. Introducing the caffeine-dependent switches to regulate CAR activity*

In vitro transcription and electroporation of mRNA for CAR experiments

4 different architectures of split CARs consisting of two chains were investigated per acVHH variant where both the first and second chain of the split CAR is either monomeric or dimeric. A dimeric first CAR chain entails an FMC63 scFv followed by a linker followed by a Flag tag followed by a CD8a hinge and transmembrane domain followed by a 4-1BB co-stimulatory domain followed by a linker followed by acVHH dfM1-1 or dfM1-2 (SEQ ID NO:34 where acVHH is dfM1-1). The only difference for a monomeric first CAR chain is the mutation of the two cysteine residues in the CD8a hinge to serine residues (SEQ ID NO:35). A dimeric second chain entails a DAP10 extracellular domain followed by a CD8 transmembrane domain followed by 2 amino acids followed by a 4-1BB co-stimulatory domain followed by a linker followed by acVHH V104D followed by a linker followed by CD3z signaling domain followed by a linker followed by mCherry (SEQ ID NO:36). A monomeric second chain entails a Strep II tag followed by a 1xG4S linker followed by a CD8a hinge, in which both cysteines were mutated to serines, followed by a CD8a transmembrane domain followed by 4-1BB co-stimulatory domain followed by a linker followed by acVHH V104D followed by a linker followed by CD3z signaling domain (SEQ ID NO:37). All constructs were generated by Gibson assembly, and the in vitro transcriptions and electroporations were carried out as described above. Primary human T cells were used as effector cells and were electroporated with the described mRNA constructs. Mock T cells are primary human T cells electroporated with no mRNA.

Jurkat T cells were electroporated with either mRNA encoding CD19 antigen (SinoBiological, RefSeq BC006338) to generate the target cells or no mRNA for the mock control.

After electroporation, the cells were incubated for 6 h at 37°C. The cells were then counted and seeded at an effector to target ratio of 2:1 with 30,000 effector T cells in phenol free media, further supplemented with either 0.5 μM AP21967 (for FKBP/FRB dimerization - Takara), or 50 μM caffeine, or only medium. The cells were co-cultured for 4 h at 37°C, then 150 μg/mL luciferin (Xenolight D-luciferin, Perkin Elmer) was added to each well and luminescence was measured after 20 min with an ENSPIRE Multimode plate reader (Perkin Elmer). Specific lysis was calculated according to the formula:

$$\%\,\text{specific lysis} = 100 - \frac{(\text{RLU target} + \text{electroporated T cells})}{(\text{RLU target} + \text{mock T cells})}\ \text{x}\ 100$$

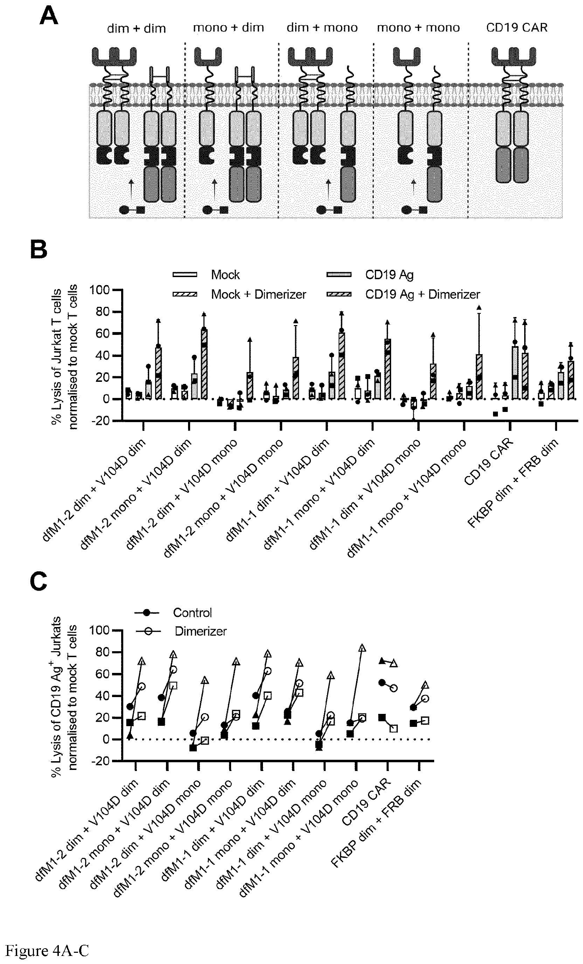

Figure 4A shows a schematic of the different CAR structures.

Figure B shows lysis of Jurkat T cells expressing or not expressing the CD19 antigen and co-cultured with or without dimerizer (0.5 μM AP21967 for FKBP/FRB dimerization or 50 μM caffeine) as indicated (n = 3, average ± standard deviation, 3 different T cell donors are represented with different symbols). The FKBP/FRB-split CAR system used is adapted from Wu et al., (Wu et al., Science. 2015; 350, SEQ ID NO:38 for chain 1 and SEQ ID NO:39 for chain 2) in which both CAR chains are dimeric. A "direct" CD19-specific CAR, i.e. without integrated on-switch domains, was used as benchmark (SEQ ID NO:40). The data in this figure clearly show a caffeine-dependent cytotoxic activity of these split CAR-expressing T cells, but only when being co-incubated with CD19-positive Jurkat target cells, demonstrating that the cytotoxic activity is dependent on the antigen on the target cell surface and that it can be regulated by administration of the small molecule caffeine.

Figure 4C shows a different representation of the data in Figure 4B by comparing only lysis of CD19-positive Jurkat T cells co-cultured with or without dimerizer.

Lentivirus production and transduction of primary human T cells

The nucleotide sequence encoding the monomeric CAR chain 1 (SEQ ID NO:34) containing dfM1-1 was fused to the nucleotide sequence encoding the dimeric CAR chain 2 (SEQ ID NO:36) or to the nucleotide sequence encoding monomeric CAR chain 2 (SEQ ID NO:37) via a furin cleavage site and 2A sequence (SEQ ID NO:41) and cloned into a third generation lentiviral expression vector with puromycin resistence gene (pCDH, System Biosciences). This plasmid, along with second-generation viral packaging plasmids (psPAX2 and pMD2.G, Addgene plasmids #12260 and #12259), was co-transfected in Lenti-X 293T cells (Takara) using the PureFection Transfection Reagent (System Biosciences) according to instructions provided by the manufacturer. Supernatants were collected on day 2 and 3 following transfection and concentrated 100-fold using Lenti-X Concentrator (Takara) according to the manufacturer's instructions. Viral suspensions were resuspended in RPMI-1640 medium supplemented with 10% FCS and 1% penicillin-streptomycin and frozen at -80 °C.

Primary human T cells were thawed and activated with αCD3/αCD28 beads (Thermo Scientific) on day 0 in AIMV++++ medium, i.e., AIMV medium (Thermo Scientific) supplemented with 2% Octaplas, 2.5% HEPES, 1% L-Glutamine and 200 U/mL recombinant human IL-2. On day 1, 100,000 T cells were seeded in retronectin (Takara) coated plates and transduced with thawed viral supernatant (diluted 1:2). Transduced cells were treated with 1 μg/mL puromycin on day 4 for 2 days and further expanded in AIMV++++ medium. Mock T cells were neither transduced nor selected with puromycin but otherwise treated the same.

Cytokine production measured by enzyme-linked immunosorbent assay (ELISA)

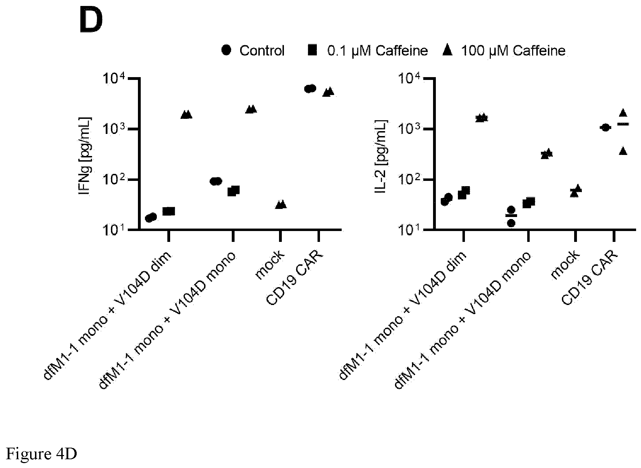

On day 12, transduced T cells were co-cultured with Raji target cells at an E:T ratio of 2:1 in the presence of either medium, or medium supplemented with 0.1 μM or 100 μM caffeine overnight at 37°C. Supernatants of triplicate wells were pooled and stored at -80°C. IFN-γ and IL-2 secretion was quantified by ELISA using kits from Biolegend (ELISA MAX Deluxe Set Human IFN-γ and ELISA MAX Deluxe Set Human IL-2, respectively), according to the manufacturer's instructions. Figure 4D shows IFN-γ and IL-2 secretion (n=1, technical duplicates are represented). These data again confirm the strong dependence of the function of these CAR T cells on the presence of caffeine. Thus, incorporation of these exemplary chemically-induced heterodimerizing systems according to the present invention into split CARs enables regulation of CAR T cell function by administration of the small molecule caffeine.

**Claims**

1. A method of creating a chemically-induced heterodimerizing system having three different components that form a ternary complex by amendment of a chemically induced homodimerizing system, wherein said chemically induced homodimerizing system comprises two components for the homodimerization, wherein the antigen binding domain comprising SEQ ID NO:1 (AB0) is the first component and a small molecule is the second component of the homodimerization, and wherein said AB0 and said small molecule form a complex of AB0/AB0/small molecule, the method comprising

    a) modifying the antigen binding domain AB0
    b) selecting a first antigen binding domain (AB 1) with a first modification from modified antigen binding domains of step a), wherein said first modification results in an impairment or a loss of said homodimerization, thereby creating said AB 1 as the first component of the heterodimerizing system
    c) modifying again the antigen binding domain AB0
    d) selecting a second antigen binding domain (AB2) with a second modification from modified antigen binding domains of step c), wherein said second modification results in a heterodimerization of AB2 with AB 1 in the presence of the small molecule, and wherein said first modification and said second modification are different modifications, thereby creating said AB2 as the second component of the heterodimerizing system
    e) expressing said AB 1 as a fusion protein with a first effector molecule and expressing said AB2 as a fusion protein with a second effector molecule,

        wherein said small molecule is the third component of the heterodimerizing system, and wherein the presence of all three components of said heterodimerizing system allows to form a ternary complex of AB 1/AB2/small molecule,
        and wherein said small molecule is a derivative of xanthine.

2. The method of claim 1, wherein said ternary complex of said 3 components is **characterized by** a $K_D$ value of 50 μM or below, wherein this $K_D$ defines the affinity between AB 1 and AB2 in the presence of said small molecule.

3. The method of claim 1 or 2, wherein the binding between AB 1 and the small molecule in the absence of AB2 is either not detectable by SPR or only shows low affinity with a $K_D$ value above 20 μM, if the small molecule is caffeine.

4. The method of claims 1 to 3, wherein said first modification comprises introducing at least one mutation into the area of the interface responsible for the homodimerization of AB0, wherein said interface responsible for the homodimerization of AB0 is defined as the amino acid positions G29 to V64 and K87 to W109 in SEQ ID NO:1, determined by analysis of the crystal structure 6QTL, and wherein said second modification comprises mutating regions in AB0 that are with regard to their position in spatial proximity to the first modification in AB1, when said ternary complex is built, as judged from the homodimeric complex structure (PDB-ID 6QTL), wherein the C alpha atom of the second modification, or at least one of the C alpha atoms of the second modification if this modification comprises more than one mutation, is located within 30 Å from the C alpha atom of any of the mutated position(s) of the first modification in AB1.

5. The method of claim 4, wherein said first modification that comprises introducing at least one mutation into the area of the interface responsible for the homodimerization of AB0 is an amino acid substitution to a hydrophilic amino acid or an amino acid insertion of a hydrophilic amino acid.

6. The method of claims 1 to 5, wherein said first modification comprises the amino acid replacement V104D or V104E in SEQ ID NO: 1 (AB0), resulting in an antigen binding domain (AB1) having at least 90% identity with the amino acid sequence as set forth in SEQ ID NO:2, if said amino acid replacement is V104D, or having at least 90% identity with the amino acid sequence as set forth in SEQ ID NO:8, if the amino acid replacement is V104E.

7. The method of claims 1 to 6, wherein said first modification results in a reduced binding between AB1 and AB0 in the presence of said small molecule, when compared to the binding in said homodimerization, e.g. in a protein complementation assay, and wherein said reduction of binding signal is at least 2-fold.

**8.** The method of claims 1 to 7, wherein said first modification is achieved by mutation of at least one amino acid that is located in close proximity to the second antigen binding domain in the homodimeric complex of AB0/AB0/caffeine (PDB-ID 6QTL), wherein the C alpha atom of this amino acid is located within 30 Å from any of the C alpha atoms of the other antigen binding domain in this homodimeric complex of AB0/AB0/caffeine.

**9.** A heterodimerizing system comprising

a) a first component that is a first protein comprising

i) a first antigen binding domain AB1, and
ii) a first effector molecule,

b) a second component that is a second protein comprising

i) a second antigen binding domain AB2, and
ii) a second effector molecule,

c) a third component, wherein said third component is a small molecule,

wherein the presence of all three components of said heterodimerizing system allows to form a ternary complex of AB1/AB2/small molecule,
and wherein said small molecule is a derivative of xanthine,
wherein said ternary complex of said three components is **characterized by** a $K_D$ value of 50 μM or below, wherein this $K_D$ defines the affinity between AB1 and AB2 in the presence of said small molecule, and wherein the binding between AB1 and the small molecule in the absence of AB2 is either not detectable by SPR or only shows low affinity with a $K_D$ value above 20μM if the small molecule is caffeine.

**10.** The heterodimerizing system of claim 9, wherein said heterodimerizing system is obtained by the method of any one of claim 1 to 8.

**11.** The heterodimerizing system of claim 9 or 10, wherein

A) said first antigen binding domain comprises an amino acid sequence having at least 90% identity with SEQ ID NO:2 with the proviso that amino acid position 104 is not modified, and wherein said second antigen binding domain comprises an amino acid sequence having at least 75%, at least 80% or at least 85% identity with SEQ ID NO:1 with the proviso that the amino acid position 104 of SEQ ID NO:1 is not modified, or
B) said first antigen binding domain comprises an amino acid sequence having at least 90% identity with SEQ ID NO:8 with the proviso that amino acid position 104 is not modified, and wherein said second antigen binding domain comprises an amino acid sequence having at least 75%, at least 80% or at least 85% identity with SEQ ID NO:1 with the proviso that the amino acid position 104 of SEQ ID NO:1 is not modified,
and wherein said small molecule is a derivative of xanthine.

**12.** The heterodimerizing system of claim 11, wherein for said second antigen binding domain of A) or B) the amino acid sequence set forth in SEQ ID NO:1 comprises the modifications:

a) amino acid replacements T50P, V51V, G52A, W53L, S54G, S55L, G56Q and/or I57S,
b) amino acid replacements T50Q, V51Q, G52R, S54L, S55F, G56R and/or I57R,
c) amino acid replacements C22A, S25Y, R27K, T50P, V51V, G52A, W53L, S54G, S55L, G56Q, I57S, C96A, T97A and/or S118T, or
d) amino acid replacements Q1E, C22A, S25Y, T50P, V51V, G52A, W53L, S54G, S55L, G56Q, I57S, C96A and/or T97A.

**13.** The heterodimerizing system of claim 9 to 12, wherein said first antigen binding domain AB1 comprises or consists of SEQ ID NO:2, and wherein said second antigen binding domain AB2 comprises or consists of SEQ ID NO:4, SEQ ID NO:5, SEQ ID NO:6 or SEQ ID NO:7 and wherein said small molecule is a derivative of xanthine.

**14.** The heterodimerizing system of claim 9 to 13, wherein said small molecule is caffeine.

**15.** The heterodimerizing system of claim 9 to 14, wherein said first and/or second effector molecule is an intracellular portion of a receptor, a transmembrane receptor that comprises one or several extracellular antigen binding domain(s), a transmembrane protein that comprises one or several intracellular signaling domain(s), a transmembrane receptor that comprises one or several extracellular antigen binding domain(s) and one or several intracellular signaling domain(s), an intracellular receptor, an extracellular protein, a caspase, a kinase, an enzyme or a protease.

A
SEC V104D
SEC F47D
SEC T50E
SEC F47K
mAU (280nm)
volume (mL)
B
Response (RU)
Caffeine [mM]
K_D: 72.36 mM
C
run 1
run 2
run 3
V104D binding [RU]
time [s]
80 mM
40 mM
20 mM
10 mM
D
gMFI binding to soluble V104D
M1
V104D
control
V104D
Caffeine
V104D + Caffeine
E
gMFI binding to soluble WT
control
WT
Caffeine
WT + Caffeine


Figure 1A-E

A
V104D point mutation
+
acVHH library with 8 NNK positions
construction of yeast display library diversity $10^8$
2 bead selection rounds diversity $10^4$
8 FACS selection rounds
2 variants: M1 and M2
B
V104D
8NNK
C
TAG
V104D
CAFFEINE
8NNK
myc
HA
Aga 2
Aga 1
Yeast cell
D
8 NNK library
2 x Positive sort
1 x Negative sort
1 x Positive sort
High
Low
1 x Negative sort
3 x Positive sort
- 1 x Stability sort
48°C heat shock, 10min
- 1 x Affinity sort
20nM V104D
Sequencing
Sequencing
F
gMFI full length display
3500
3000
2500
2000
1500
1000
500
0
M1
M2
WT

Figure 2A-D, F

E
Positive sort
Negative sort
acVHH M1
acVHH M2
control
caffeine
High
Low
Binding to V104D
HA-display of the variant
G
gMFI binding to V104D
30000
20000
10000
0
M1
M2
WT
Adenine
Guanine
Theobromine
Paraxanthine
Theophylline
Caffeine
control
H
Normalised binding signal
1.0
0.8
0.6
0.4
0.2
0.0
0.1
1
10
100
1000
V104D [nM]

Figure 2E, G, H

A
C22
C96
acVHH WT
B
full length display normalised to M1
1.5
1.0
0.5
0.0
C
gMFI binding to V104D
8000
6000
4000
2000
0
Control
Caffeine
D
epPCR on a mixture of M1ΔAA, ΔAV, ΔVA, ΔWP
construction of yeast display library diversity $10^8$
2 bead selection rounds diversity $10^4$
11 FACS selection rounds
2 variants: dfM1-1 dfM1-2
E
M1Δ library
epPCR after bead selection n°2
epPCR after sort n°2
2 x Positive sort
2 x Positive sort
2 x Negative sort
Not working
3 x Positive sort
high
low
1 x Positive sort
1 x Negative sort
1 x Positive sort
1 x Negative sort
2 x Positive sort
Sequencing
Only de novo C22-C96
Sequencing
2 x Positive sort
1 x Negative sort
6 x Positive sort
Sequencing
2 variants

Figure 3A-E

F
gMFI binding to V104D
M1
dfM1-1
dfM1-2
Adenine
Guanine
Theobromine
Paraxanthine
Theophylline
Caffeine
PBSA
G
gMFI full length display normalised to M1
M1
dfM1-1
dfM1-2
H
Normalised binding signal
V104D [nM]
I
Normalised binding signal
WT / WT
V104D / M1
V104D / dfM1-1
V104D / dfM1-2
Caffeine [µM]
J
Binding normalised to WT + Caffeine
Control
Caffeine
V104D / -
V104D / V104D
V104D / M1
V104D / M2
V104D / dfM1-1
V104D / dfM1-2
WT / WT
K
RLU (background subtracted)
WT / WT
V104D / dfM1-2
V104D / dfM1-1
V104D / M1
V104D / V104D
V104D / WT
Caffeine [µM]
L
RLU (background subtracted)
WT / WT
V104D / dfM1-2
V104D / dfM1-1
V104D / M1
V104D / V104D
V104D / WT
Theobromine [µM]

Figure 3F-L

M
WT / WT Caffeine
V104D / dfM1-2 Caffeine
V014D / dfM1-1 Caffeine
V104D / M1 Caffeine
Binding signal normalised
1.0
0.8
0.6
0.4
0.2
0.0
10-3
10-2
10-1
10^0
10^1
10^2
10^3
Caffeine [µM]
N
O
WT QVQLVESGGGLVQAGGSLRLS C TASGRTGTIYSMAWFRQAPGKEREFLA TVGWSSGI TYYMDSVKGRFTISRDKGKNTVYLQMDSLKPEDTAVYY C TATRAYSVGYDYWGQGTQVTVSS
V104D QVQLVESGGGLVQAGGSLRLS C TASGRTGTIYSMAWFRQAPGKEREFLA TVGWSSGI TYYMDSVKGRFTISRDKGKNTVYLQMDSLKPEDTAVYY C TATRAYSDGYDYWGQGTQVTVSS
M2 QVQLVESGGGLVQAGGSLRLS C TASGRTGTIYSMAWFRQAPGKEREFLA QQRWLFRR TYYMDSVKGRFTISRDKGKNTVYLQMDSLKPEDTAVYY C TATRAYSVGYDYWGQGTQVTVSS
M1 QVQLVESGGGLVQAGGSLRLS C TASGRTGTIYSMAWFRQAPGKEREFLA PVALGLQS TYYMDSVKGRFTISRDKGKNTVYLQMDSLKPEDTAVYY C TATRAYSVGYDYWGQGTQVTVSS
dfM1-1 QVQLVESGGGLVQAGGSLRLS A TAYGKTGTIYSMAWFRQAPGKEREFLA PVALGLQS TYYMDSVKGRFTISRDKGKNTVYLQMDSLKPEDTAVYY A AATRAYSVGYDYWGQGTQVTVTS
dfM1-2 EVQLVESGGGLVQAGGSLRLS A TAYGRTGTIYSMAWFRQAPGKEREFLA PVALGLQS TYYMDSVKGRFTISRDKGKNTVYLQMDSLKPEDTAVYY A AATRAYSVGYDYWGQGTQVTVSS
* * * * ******** * * * *

| | $EC_{50}$ (µM; mean ± S.D.) | |
|---|---|---|
| | $EC_{50,YSD}$ | $EC_{50,NanoBIT}$ |
| WT / WT | 4,3 ± 1,3 | 2,5 |
| V104D / M1 | 104,4 ± 50,5 | 64,7 |
| V104D / dfM1-1 | 83,0 ±23,8 | 9,1 |
| V104D / dfM1-2 | 124,2 ± 36,7 | 10 |

| | $K_D$ (nM; mean ± S.D.) | |
|---|---|---|
| | Yeast display | |
| | + Caffeine | - Caffeine |
| V104D / M1 | 30,0 ± 3,7 | n.a. |
| V104D / dfM1-1 | 18,8 ± 2,6 | n.a. |
| V104D / dfM1-2 | 21,8 ± 2,6 | n.a. |

Figure 3M-O

A
dim + dim
mono + dim
dim + mono
mono + mono
CD19 CAR
B
Mock
Mock + Dimerizer
CD19 Ag
CD19 Ag + Dimerizer
% Lysis of Jurkat T cells normalised to mock T cells
dfM1-2 dim + V104D dim
dfM1-2 mono + V104D dim
dfM1-2 dim + V104D mono
dfM1-2 mono + V104D mono
dfM1-1 dim + V104D dim
dfM1-1 mono + V104D dim
dfM1-1 dim + V104D mono
dfM1-1 mono + V104D mono
CD19 CAR
FKBP dim + FRB dim
C
Control
Dimerizer
% Lysis of CD19 Ag+ Jurkats normalised to mock T cells

Figure 4A-C

D
Control
0.1 µM Caffeine
100 µM Caffeine
IFNg [pg/mL]
IL-2 [pg/mL]
10^4
10^3
10^2
10^1
dfM1-1 mono + V104D dim
dfM1-1 mono + V104D mono
mock
CD19 CAR

Figure 4D

Europäisches
Patentamt
European
Patent Office
Office européen
des brevets

# EUROPEAN SEARCH REPORT

Application Number
EP 23 17 6793

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2017/070554 A1 (HUTCHINSON FRED CANCER RES [US]) 27 April 2017 (2017-04-27)<br>* paragraph [0031] - paragraph [0036] *<br>* paragraph [0157] - paragraph [0160]; figure 2 * | 1-15 | INV.<br>C07K16/44<br>A61K39/00 |
| Y | BOJAR DANIEL ET AL: "Caffeine-inducible gene switches controlling experimental diabetes",<br>NATURE COMMUNICATIONS, [Online]<br>vol. 9, no. 1, 19 June 2018 (2018-06-19), XP055786426,<br>DOI: 10.1038/s41467-018-04744-1<br>Retrieved from the Internet:<br>URL:http://www.nature.com/articles/s41467-018-04744-1><br>[retrieved on 2023-10-20]<br>* figure 1; example all * | 1-15 | |

TECHNICAL FIELDS SEARCHED (IPC)

C07K
A61K

The present search report has been drawn up for all claims

1

| Place of search | Date of completion of the search | Examiner |
|---|---|---|
| Munich | 20 October 2023 | Fellows, Edward |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 17 6793

This annex lists the patent family members relating to the patent documents cited in the above-mentioned European search report. The members are as contained in the European Patent Office EDP file on
The European Patent Office is in no way liable for these particulars which are merely given for the purpose of information.

20-10-2023

| Patent document cited in search report | Publication date | Patent family member(s) | Publication date |
|---|---|---|---|
| WO 2017070554 A1 | 27-04-2017 | CA 3002903 A1 | 27-04-2017 |
| | | EP 3365442 A1 | 29-08-2018 |
| | | ES 2923755 T3 | 30-09-2022 |
| | | HK 1258313 A1 | 08-11-2019 |
| | | US 2018319870 A1 | 08-11-2018 |
| | | US 2021198646 A1 | 01-07-2021 |
| | | WO 2017070554 A1 | 27-04-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014127261 A1
- WO 2018213848 A1
- WO 2017070554 A
- WO 2006036882 A2
- WO 2020070289 A1
- US 9233125 B2
- WO 2017091546 A


### Non-patent literature cited in the description


- **RIVERA et al.** *Nature medicine,* 1996, vol. 2 (9), 1028-32
- **CHOI et al.** *Science,* 1996, vol. 273 (5272), 239-42
- **LADENSON, R. C. et al.** *Analytical Chemistry,* 2006, vol. 78, 4501-4508
- **BOJAR, D et al.** *Nat. Commun.,* 2018, vol. 9, 2318
- **FRANCO, E. J. et al.** *Journal of Chromatography B,* 2010, vol. 878, 177-186
- **LESNE, J et al.** *Scientific reports,* 2019, vol. 9, 1840
- **TEEKACHUNHATEAN, S. et al.** *ISRN Pharmacol.,* 2013, vol. 2013, 147238
- **ROBERTSON, D. et al.** *N Engl J Med,* 1978, vol. 1978 (298), 181-186
- **CORTI, R. et al.** *Circulation,* 2002, vol. 106, 2935-2940
- **WEBER, E.W. et al.** *Science,* 2021, vol. 372 (6537), 1786
- **DIXON et al.** *ACS Chem Biol.,* 19 February 2016, vol. 11 (2), 400-8
- **CHAO et al.** *Nat Protoc.,* 2006, vol. 1 (2), 755-768
- **ANGELINI et al.** *Methods Mol Biol.,* 2015, vol. 1319, 3-36
- **CHEN et al.** *Methods Enzymol.,* 2013, vol. 523, 303-26
- **ZAJC, C.U. et al.** *FEBS J.,* 2021, vol. 288 (7), 2103-2118
- **TRAXLMAYR et al.** *J Biol Chem.,* 21 October 2016, vol. 291 (43), 22496-22508
- **ZAJC et al.** *Methods Mol Biology.,* 2022, vol. 2491, 155-173
- **ZAJC et al.** *Methods Mol Biology,* 2022, vol. 2491, 155-173
- **WU et al.** *Science,* 2015, 350